# EUROPEAN PATENT APPLICATION

(11) **EP 4 506 360 A1**
(43) Date of publication of application: **12.02.2025**
(21) Application number: 23778428.5
(22) Date of filing: 31.03.2023
(51) Int. Cl.: C07K 16/28, C12N 15/13, C12N 15/63, C12N 5/10, A61K 39/395, A61P 35/00

(54) **ANTI-PVRIG ANTIBODY, PHARMACEUTICAL COMPOSITION THEREOF AND USE THEREOF**

(30) Priority: 02.04.2022 CN 202210343374
(71) Applicant: Biotheus Inc., Tangjiawan Town, Xiangzhou District Zhuhai, Guangdong 519080 (CN)
(72) Inventor: ZHAI, Tianhang, Zhuhai, Guangdong 519080 (CN); DAI, Shuang, Zhuhai, Guangdong 519080 (CN); HUANG, Weifeng, Zhuhai, Guangdong 519080 (CN); TSUN, Andy, Zhuhai, Guangdong 519080 (CN); BUKOWSKI, Jon, Zhuhai, Guangdong 519080 (CN); SCHUTZ, Kevin, Zhuhai, Guangdong 519080 (CN); HEMMERLEIN, Megan, Zhuhai, Guangdong 519080 (CN)
(74) Representative: Ghirardi, Valeria
(86) International application number: PCT/CN2023/085357
(87) International publication number: WO 2023/186063

(57) **Abstract**

Provided in the present invention is an anti-PVRIG antibody or an antigen-binding fragment thereof, wherein the antibody comprises a heavy chain variable region and a light chain variable region. The heavy chain variable region comprises HCDR1 to HCDR3, and the light chain variable region comprises LCDR1 to LCDR3. In the heavy chain variable region of the antibody, the amino acid sequence of HCDR1 is as shown in SEQ ID NO: 9, the amino acid sequence of HCDR2 is as shown in SEQ ID NO: 10, and the amino acid sequence of HCDR3 is as shown in SEQ ID NO: 11; and in the light chain variable region of the antibody, the amino acid sequence of LCDR1 is as shown in SEQ ID NO: 12, the amino acid sequence of LCDR2 is as shown in SEQ ID NO: 13, and the amino acid sequence of LCDR3 is as shown in SEQ ID NO: 14.

## Description

### Technical Field

The present invention belongs to the field of biomedicine and relates to an anti-PVRIG antibody, pharmaceutical composition thereof and use thereof.

### Background Art

Poliovirus receptor related immunoglobulin domain-containing protein (PVRIG, also known as CD112R) is a member of the PVR family with an IgV structural domain in the extracellular region and an Immunoreceptor tyrosine-based inhibitor motif (ITIM) in the intracellular region, and is a novel immunosuppressive receptor expressed on natural killer (NK) cells and T cells.

Poliovirus receptor-related 2 (PVRL2, CD112, Nectin-2) is an adhesion molecule involved in cell-cell junctions and is overexpressed in several cancers. PVRL2 has been identified as the only functional ligand for PVRIG that binds to PVRIG with high affinity and inhibits immune activation of T and NK cells. PVRL2 is also a ligand for co-activation receptor DNAX accessory molecule-1 (DNAM-1, CD226) and binds weakly to another inhibitory receptor-T cell immunoreceptor with Ig and ITIM domain (TIGIT). Therefore, similar to the immunosuppressive mechanism of TIGIT, a member of the same family, PVRIG can also act as an immunosuppressor by blocking the activation signals transmitted by CD226 through competitive binding to its ligand PVRL2.

PVRIG or its ligand PVRL2 is highly expressed in lung, kidney, endometrial, breast and skin cancers and their tumor microenvironments, and PVRIG was also highly expressed on NK cells from prostate cancer patients (Whelan S, et al. Cancer Immunol Res. 2019;7(2):257-268.). Additionally, PVRIG is co-expressed with the exhaustion markers TIGIT and PD-1 on CD8+ T cells, suggesting that PVRIG expression has certain tumor specificity and may be associated with the activated/exhausted state of TILs (Whelan S, et al. Cancer Immunol Res. 2019;7(2):257-268.). WO2021180205A1 reported that anti-PVRIG antibodies can be used for the treatment of lung cancer, breast cancer, ovarian cancer, kidney cancer, gastric cancer, endometrial cancer and head and neck cancer.

Studies in preclinical mouse tumor models have shown that PVRIG depletion or blockade restores T/NK cell activity and promotes anti-tumor immune responses. CD8+ T cells isolated from Pmel-1 TCR-CD112R-deficient mice exhibit enhanced degranulation and cytokine secretion. MC38 tumor growth is slower in CD112R-deficient mice compared to wild-type mice, and CD8⁺ TILs display enhanced immune effects and an upregulated transcriptome profile of inflammation/cytotoxicity-related genes. The literature also reported that PVRIG antibody or PVRIG gene knockout mice in combination with PD-L1 antibody in MC38 and CT26 mouse tumor models provided better therapeutic efficacy than PD-L1 antibody alone (Murter B, et al. Cancer Immunology Research, 2019.). Feng Xu et al. have demonstrated that blocking the interaction of PVRIG with CD112 using antibodies enhances the cytotoxicity of NK cells against tumor cells, and in combination with a TIGIT-blocking monoclonal antibody further enhances the trastuzumab-mediated killing effect of breast cancer cells by NK cells (Xu F, et al. Cancer Immunology Immunotherapy Cii, 2017.). In a knockout mouse model, PVRIG antibody reduced tumor growth in TIGIT-/- mice, and tumor growth control was further improved in PVRIG and TIGIT double knockout mice compared to single gene knockout mice (RJ Sullivan, et al, ASCO-SITC Clinical Immuno-Oncology Symposium, 2020).

Preclinical data suggested that PVRIG antibody can effectively stimulate anti-tumor immune responses in certain cancers, and it has been found to have some synergistic effects with PD-1/L1 and TIGIT antibodies, which can enhance the function of T/NK cells and improve anti-tumor response and slow tumor growth. PVRIG as a therapeutic target is expected to improve the therapeutic efficacy of existing antibody drugs (e.g., anti-TIGIT and anti-PD-1/L1 antibodies), and provide advantages over other drug targets and combination therapies in the clinic. COM701 developed by Compugen, is the world's first clinically approved PVRIG antibody, and is currently in a Phase I trial in combination with a TIGIT monoclonal antibody (COM902) and has entered into a collaboration with BMS to develop a triple combination Phase I trial of COM701+BMS-986207+nivolumab. In addition, GSK4381562 from Surface Oncology licensed to GSK and JS009 from Junshi Biosciences in China, which have filed clinical applications by the end of 2021, and Amgen has also developed the PVRIG monoclonal antibody, which is currently in the preclinical development.

Therefore, there is a need in this field to develop new high-affinity, selective and biologically active anti-PVRIG antibodies for the treatment of diseases, especially cancer.

### Contents of the present invention

After intensive research, the inventors of the present application obtained a novel anti-PVRIG antibody. The inventors surprisingly found that the anti-PVRIG antibody of the present invention exhibits excellent affinity and biological activity, with potential for anti-tumor effects. This has led to the following inventions:
One aspect of the present invention relates to an anti-PVRIG antibody or an antigen-binding fragment thereof, which comprises:
wherein, the antibody comprises a heavy chain variable region comprising HCDR1 to HCDR3 and a light chain variable region comprising LCDR1 to LCDR3, wherein:
the heavy chain variable region of the antibody comprises,
HCDR1 with the amino acid sequence as set forth in SEQ ID NO:9, HCDR2 with the amino acid sequence as set forth in SEQ ID NO:10 and HCDR3 with the amino acid sequence as set forth in SEQ ID NO:11,
HCDR1 with the amino acid sequence as set forth in SEQ ID NO:15, HCDR2 with the amino acid sequence as set forth in SEQ ID NO:16 and HCDR3 with the amino acid sequence as set forth in SEQ ID NO:17,
HCDR1 with the amino acid sequence as set forth in SEQ ID NO:21, HCDR2 with the amino acid sequence as set forth in SEQ ID NO:22 and HCDR3 with the amino acid sequence as set forth in SEQ ID NO:23, or
HCDR1 with the amino acid sequence as set forth in SEQ ID NO:27, HCDR2 with the amino acid sequence as set forth in SEQ ID NO:28 and HCDR3 with the amino acid sequence as set forth in SEQ ID NO:29;
   and
the light chain variable region of the antibody comprises,
LCDR1 with the amino acid sequence as set forth in SEQ ID NO:12, LCDR2 with the amino acid sequence as set forth in SEQ ID NO:13 and LCDR3 with the amino acid sequence as set forth in SEQ ID NO:14,
LCDR1 with the amino acid sequence as set forth in SEQ ID NO:18, LCDR2 with the amino acid sequence as set forth in SEQ ID NO:19 and LCDR3 with the amino acid sequence as set forth in SEQ ID NO:20,
LCDR1 with the amino acid sequence as set forth in SEQ ID NO:24, LCDR2 with the amino acid sequence as set forth in SEQ ID NO:25 and LCDR3 with the amino acid sequence as set forth in SEQ ID NO:26, or
LCDR1 with the amino acid sequence as set forth in SEQ ID NO:30, LCDR2 with the amino acid sequence as set forth in SEQ ID NO:31 and LCDR3 with the amino acid sequence as set forth in SEQ ID NO:32.

In some embodiments of the present invention, the anti-PVRIG antibody or antigen-binding fragment thereof is provided, wherein,
the heavy chain variable region of the antibody comprises HCDR1 with the amino acid sequence as set forth in SEQ ID NO:9, HCDR2 with the amino acid sequence as set forth in SEQ ID NO:10 and HCDR3 with the amino acid sequence as set forth in SEQ ID NO:11, and, the light chain variable region of the antibody comprises LCDR1 with the amino acid sequence as set forth in SEQ ID NO: 12, LCDR2 with the amino acid sequence as set forth in SEQ ID NO: 13 and LCDR3 with the amino acid sequence as set forth in SEQ ID NO:14;
the heavy chain variable region of the antibody comprises HCDR1 with the amino acid sequence as set forth in SEQ ID NO:15, HCDR2 with the amino acid sequence as set forth in SEQ ID NO:16 and HCDR3 with the amino acid sequence as set forth in SEQ ID NO:17, and, the light chain variable region of the antibody comprises LCDR1 with the amino acid sequence as set forth in SEQ ID NO: 18, LCDR2 with the amino acid sequence as set forth in SEQ ID NO: 19 and LCDR3 with the amino acid sequence as set forth in SEQ ID NO:20;
the heavy chain variable region of the antibody comprises HCDR1 with the amino acid sequence as set forth in SEQ ID NO:21, HCDR2 with the amino acid sequence as set forth in SEQ ID NO:22 and HCDR3 with the amino acid sequence as set forth in SEQ ID NO:23, and, the light chain variable region of the antibody comprises LCDR1 with the amino acid sequence as set forth in SEQ ID NO:24, LCDR2 with the amino acid sequence as set forth in SEQ ID NO:25 and LCDR3 with the amino acid sequence as set forth in SEQ ID NO:26;
   or
the heavy chain variable region of the antibody comprises HCDR1 with the amino acid sequence as set forth in SEQ ID NO:27, HCDR2 with the amino acid sequence as set forth in SEQ ID NO:28 and HCDR3 with the amino acid sequence as set forth in SEQ ID NO:29, and, the light chain variable region of the antibody comprises LCDR1 with the amino acid sequence as set forth in SEQ ID NO:30, LCDR2 with the amino acid sequence as set forth in SEQ ID NO:31 and LCDR3 with the amino acid sequence as set forth in SEQ ID NO:32.

The variable regions of the light chain and the heavy chain determine the binding to antigen; the variable region of each chain comprises three hypervariable regions, called complementarity determining regions (CDRs), of which the CDRs of the heavy chain (H) include HCDR1, HCDR2, and HCDR3, and the CDRs of the light chain (L) include LCDR1, LCDR2, and LCDR3. In some embodiments of the present invention, HCDR1-HCDR3 and LCDR1-LCDR3 are defined or numbered according to the United States Patent Publication US20210380669A1 or the literature Lu et al (Deamidation and isomerization liability analysis of 131 clinical-stage antibodies, MABS, 2019, VOL. 11, NO. 1, 45-57, DOI: 10.1080/19420862.2018.1548233).

In some embodiments of the present invention, the anti-PVRIG antibody or antigen-binding fragment thereof is provided, wherein, the heavy chain variable region has an amino acid sequence selected from SEQ ID NO: 1, SEQ ID NO: 3, SEQ ID NO: 5 and SEQ ID NO: 7;
and
the light chain variable region has an amino acid sequence selected from SEQ ID NO:2, SEQ ID NO:4, SEQ ID NO:6 and SEQ ID NO:8.

In some embodiments of the present invention, the anti-PVRIG antibody or antigen-binding fragment thereof is provided, wherein,
the heavy chain variable region has an amino acid sequence as set forth in SEQ ID NO: 1, and the light chain variable region has an amino acid sequence as set forth in SEQ ID NO: 2;
the heavy chain variable region has an amino acid sequence as set forth in SEQ ID NO:3, and the light chain variable region has an amino acid sequence as set forth in SEQ ID NO:4;
the heavy chain variable region has an amino acid sequence as set forth in SEQ ID NO:5, and the light chain variable region has an amino acid sequence as set forth in SEQ ID NO:6;
   or
the heavy chain variable region has an amino acid sequence as set forth in SEQ ID NO:7, and the light chain variable region has an amino acid sequence as set forth in SEQ ID NO:8.

In some embodiments of the present invention, the anti-PVRIG antibody or antigen-binding fragment thereof is provided, wherein,
the heavy chain constant region of the antibody is Ig gamma-1 chain C region or Ig gamma-4 chain C region; the light chain constant region is Ig kappa chain C region;
preferably, the heavy chain constant region of the antibody has an amino acid sequence as set forth in SEQ ID NO: 33, SEQ ID NO: 34 or SEQ ID NO: 35, and the light chain constant region of the antibody has an amino acid sequence as set forth in SEQ ID NO: 36.

In some embodiments of the present invention, the anti-PVRIG antibody or antigen-binding fragment thereof is provided, wherein, the heavy chain constant region of the antibody comprises a L234A mutation and a L235A mutation according to the EU numbering system (abbreviated as LALA). In the present invention, unless otherwise specified, the letter before the site represents the amino acid before mutation, and the letter after the site represents the amino acid after mutation.

In some embodiments of the present invention, the anti-PVRIG antibody or antigen-binding fragment thereof is provided, wherein, the anti-PVRIG antibody or antigen-binding fragment is selected from the group consisting of Fab, Fab', F(ab')₂, Fd, Fv, dAb, a complementarity determining region fragment, a single chain antibody, a humanized antibody, a chimeric antibody and a diabody.

In some embodiments of the present invention, the anti-PVRIG antibody or antigen-binding fragment thereof is provided, wherein, the antibody comprises a non-CDR region, and the non-CDR region is derived from a species other than murine, such as from a human antibody.

In some embodiments of the present invention, the anti-PVRIG antibody or antigen-binding fragment thereof is provided, wherein the antibody binds to human PVRIG protein overexpressed on the surface of CHO cells with an EC₅₀ of less than 3nM, less than 2.9nM or less than 2.8nM; preferably, the EC₅₀ is measured by flow cytometry assay method.

Another aspect of the present invention relates to an isolated nucleic acid molecule, which encodes the anti-PVRIG antibody or antigen-binding fragment thereof according to any embodiment of the present invention.

A further aspect of the present invention relates to a vector, which comprises the isolated nucleic acid molecule of the present invention.

A further aspect of the present invention relates to a host cell, which comprises the isolated nucleic acid molecule of the present invention, or the vector of the present invention.

A further aspect of the present invention relates a conjugate, which comprises an antibody and a conjugated moiety, wherein the antibody is an anti-PVRIG antibody or an antigen-binding fragment thereof according to any one of any embodiment of the present invention, and the conjugated moiety is a detectable label; preferably, the conjugated moiety is a radioactive isotope, a fluorescent substance, a colored substance, or an enzyme.

Another aspect of the present invention relates to a kit, which comprises the anti-PVRIG antibody according to any embodiment of the present invention, or the conjugate of present invention;
preferably, the kit further comprises a second antibody that specifically recognizes the antibody; optionally, the second antibody further comprises a detectable label, such as a radioactive isotope, a fluorescent substance, a colored substance or an enzyme.

Another aspect of the present invention relates to a pharmaceutical composition, which comprises the anti-PVRIG antibody or an antigen-binding fragment according to any embodiment of the present invention, and one or more pharmaceutically acceptable excipients.

In some embodiments of the present invention, the pharmaceutical composition further comprising one or more anti-TIGIT antibodies;
preferably, the pharmaceutical composition further comprises one or more anti-PD-1 antibodies or one or more anti-PD-L1 antibodies.

In some embodiments of the present invention, the pharmaceutical composition is provided, wherein, the molar ratio of anti-PVRIG antibody or its antigen-binding fragment to anti-TIGIT antibody is from (1:5) to (5:1); for example, 1:5, 1:4, 1:3, 1:2, 1:1, 2:1, 3:1, 4:1, or 5:1;
and/or
the molar ratio of anti-PVRIG antibody or antigen-binding fragment to anti-PD-1 antibody or anti-PD-L1 antibody is from (1:5) to (5:1); for example, 1:5, 1:4, 1:3, 1:2, 1:1, 2:1, 3:1, 4:1, or 5:1.

In one or more embodiments of the present invention, the pharmaceutical composition is provided, wherein, the anti-TIGIT antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-TIGIT antibody comprises HCDR1 with the amino acid sequence as set forth in SEQ ID NO:42, HCDR2 with the amino acid sequence as set forth in SEQ ID NO:43 and HCDR3 with the amino acid sequence as set forth in SEQ ID NO:44, and, the light chain variable region of the anti-TIGIT antibody comprises LCDR1 with the amino acid sequence as set forth in SEQ ID NO:45, LCDR2 with the amino acid sequence as set forth in SEQ ID NO:46 and LCDR3 with the amino acid sequence as set forth in SEQ ID NO:47;
preferably, the heavy chain variable region of the anti-TIGIT antibody has an amino acid sequence as set forth in SEQ ID NO:40, and the light chain variable region has an amino acid sequence as set forth in SEQ ID NO:41.

In one or more embodiments of the present invention, the pharmaceutical composition is provided, wherein, the anti-PD-1 antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-PD-1 antibody comprises HCDR1 with the amino acid sequence as set forth in SEQ ID NO:50, HCDR2 with the amino acid sequence as set forth in SEQ ID NO:51 and HCDR3 with the amino acid sequence as set forth in SEQ ID NO:52, and, the light chain variable region of the anti-PD-1 antibody comprises LCDR1 with the amino acid sequence as set forth in SEQ ID NO:53, LCDR2 with the amino acid sequence as set forth in SEQ ID NO:54 and LCDR3 with the amino acid sequence as set forth in SEQ ID NO:55;
preferably, the heavy chain variable region of the anti-PD-1 antibody has an amino acid sequence as set forth in SEQ ID NO: 48, and the light chain variable region has an amino acid sequence as set forth in SEQ ID NO: 49.

In one or more embodiments of the present invention, the pharmaceutical composition is provided, wherein the unit dose of the pharmaceutical composition, calculated by the mass of the bispecific antibody contained therein, is 100mg-1000mg, 200mg-800mg, 200mg-500mg, 300mg-600mg, 400mg-500mg, or 450mg.

In some embodiments of the present invention, the pharmaceutical composition is provided, wherein the active ingredient of the pharmaceutical composition consists of an anti-PVRIG antibody or its antigen-binding fragment.

In some embodiments of the present invention, the pharmaceutical composition is provided, wherein the active ingredient of the pharmaceutical composition consists of an anti-PVRIG antibody or its antigen-binding fragment and an anti-TIGIT antibody.

In some embodiments of the present invention, the pharmaceutical composition is provided, wherein the active ingredient of the pharmaceutical composition consists of an anti-PVRIG antibody or its antigen-binding fragment, an anti-TIGIT antibody, and an anti-PD-1 antibody.

Another aspect of the present invention relates to a combination product, which comprises a first product and a second product in separate packages, wherein,
the first product comprises the anti-PVRIG antibody or antigen-binding fragment thereof according to any embodiment of the present invention or the conjugate of the present invention;
the second product comprises at least one anti-TIGIT antibody;
preferably, the combination product further comprises a third product, the third product comprising at least one anti-PD-1 antibody or at least one anti-PD-L1 antibody;
preferably, the first product, the second product and the third product also independently contain one or more pharmaceutically acceptable excipients;
preferably, the combination product further comprises a package insert.

In some embodiments of the present invention, the combination product is provided, wherein,
the molar ratio of anti-PVRIG antibody or its antigen-binding fragment to anti-TIGIT antibody is from (1:5) to (5:1); for example, 1:5, 1:4, 1:3, 1:2, 1:1, 2:1, 3:1, 4:1, or 5:1;
   and / or
the molar ratio of anti-PVRIG antibody or antigen-binding fragment to anti-PD-1 antibody or anti-PD-L1 antibody is from (1:5) to (5: 1), for example, 1:5, 1:4, 1:3, 1:2, 1:1, 2:1, 3:1, 4:1, or 5:1;

In some embodiments of the present invention, the combination product is provided, wherein, the anti-TIGIT antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-TIGIT antibody comprises HCDR1 with the amino acid sequence as set forth in SEQ ID NO:42, HCDR2 with the amino acid sequence as set forth in SEQ ID NO:43 and HCDR3 with the amino acid sequence as set forth in SEQ ID NO:44, and, the light chain variable region of the anti-TIGIT antibody comprises LCDR1 with the amino acid sequence as set forth in SEQ ID NO:45, LCDR2 with the amino acid sequence as set forth in SEQ ID NO:46 and LCDR3 with the amino acid sequence as set forth in SEQ ID NO:47;
preferably, the heavy chain variable region of the anti-TIGIT antibody has an amino acid sequence as set forth in SEQ ID NO:40, and the light chain variable region has an amino acid sequence as set forth in SEQ ID NO:41.

In some embodiments of the present invention, the combination product is provided, wherein, the anti-PD-1 antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti- PD-1 antibody comprises HCDR1 with the amino acid sequence as set forth in SEQ ID NO:50, HCDR2 with the amino acid sequence as set forth in SEQ ID NO:51 and HCDR3 as set forth in SEQ ID NO:52, and, the light chain variable region of the anti- PD-1 antibody comprises LCDR1 with the amino acid sequence as set forth in SEQ ID NO:53, LCDR2 with the amino acid sequence as set forth in SEQ ID NO:54 and LCDR3 with the amino acid sequence as set forth in SEQ ID NO:55;
preferably, the heavy chain variable region of the anti-PD-1 antibody has an amino acid sequence as set forth in SEQ ID NO: 48, and the light chain variable region has an amino acid sequence as set forth in SEQ ID NO: 49.

Another aspect of the present invention relates to a use of the antibody or antigen-binding fragment thereof according to any embodiment of the present invention, or the conjugate of the present invention in the preparation of a medicament for the treatment or prevention of a tumor;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.

The antibody or antigen-binding fragment thereof according to any embodiment of the present invention or the conjugate of the present invention for use in the treatment or prevention of a tumor;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.

Another aspect of the present invention relates to a method for treating or preventing a tumor, comprising a step of administering to a subject in need an effective amount of the antibody or antigen-binding fragment thereof according to any embodiment of the present invention or the conjugate of the present invention;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.

In one or more embodiments of the present invention, the method of treating and/or preventing the malignant tumor is provided, wherein,
the single dose of the anti-PVRIG antibody is 0.1 to 100mg, preferably 1 to10mg (e.g., 1 mg, 2 mg, 3 mg, 4 mg, 5 mg, 6 mg, 7 mg, 8 mg, 9 mg, or 10 mg), per kilogram of body weight,; alternatively, the single dosage of the anti-PVRIG antibody of the present invention is 10 to 1000mg per subject (e.g., approximately 100mg, approximately 150mg, approximately 200 mg, approximately 250 mg, approximately 300 mg, approximately 350 mg, approximately 400 mg, approximately 450 mg, approximately 500 mg, approximately 600 mg, approximately 700 mg, approximately 800 mg, approximately 900 mg, or approximately 1000 mg), preferably 50-500mg, 100-400mg, 150-300mg, 150-250mg, or 200mg, per kilogram of body weight;
preferably, the administering is performed every 3 days, 4 days, 5 days, 6 days, 10 days, 1 week, 2 weeks, or 3 weeks;
preferably, the administering is performed by the manner of intravenous drip or intravenous injection.

In some regimens, the administration of the anti-PVRIG antibody is in cycles of 2 weeks (14 days) or 3 weeks (21 days), preferably with the anti-PVRIG antibody administered intravenously on the first day (D1) of each cycle. For example, the anti-PVRIG antibody is administered at a frequency of once every two weeks (q2w) or once every three weeks (q3w).

In the present invention, unless otherwise stated, scientific and technical terms used herein have the meanings commonly understood by those skilled in the art. Moreover, the procedures of cell culture, molecular genetics, nucleic acid chemistry, and immunology laboratory used herein are routine procedures widely used in the corresponding fields. Meanwhile, in order to better understand the present invention, definitions and explanations of relevant terms are provided below.

As used herein, when referring to the amino acid sequence of PVRIG (NCBI GenBank ID: NP_076975.2), it includes the full-length PVRIG protein or its extracellular domain; it also includes fusion proteins of PVRIG, such as a fragment fused to a mouse or human IgG Fc protein fragment (mFc or hFc). However, those skilled in the art understand that in the amino acid sequence of PVRIG protein, a mutation or variation (including but not limited to substitution, deletion and/or addition) can occur naturally or be artificially introduced without affecting its biological function. Therefore, in the present invention, the term " PVRIG protein" or "PVRIG" shall include all such sequences, including the sequences shown and natural or artificial variants thereof. Moreover, when describing a sequence fragment of PVRIG protein, it includes not only the sequence fragment, but also the corresponding sequence fragment in its natural or artificial variants.

As used herein, when referring to the amino acid sequence of TIGIT (NCBI GenBank ID: NP_776160.2), it includes the full-length TIGIT protein or its functional fragment; it also includes fusion proteins of TIGIT, such as a fragment fused to a mouse or human IgG Fc protein fragment (mFc or hFc). However, those skilled in the art understand that in the amino acid sequence of TIGIT protein, a mutation or variation (including but not limited to substitution, deletion and/or addition) can occur naturally or be artificially introduced without affecting its biological function. Therefore, in the present invention, the term " TIGIT protein" or "TIGIT" shall include all such sequences, including the sequences shown and natural or artificial variants thereof. Moreover, when describing a sequence fragment of TIGIT protein, it includes not only the sequence fragment, but also the corresponding sequence fragment in its natural or artificial variants.

As used herein, the term "EC₅₀" refers to a concentration for 50% of maximal effect, which refers to a concentration that causes 50% of maximum effect.

As used herein, the term "antibody" refers to an immunoglobulin molecule usually composed of two pairs of polypeptide chains, each pair having a "light" (L) chain and a "heavy" (H) chain. Antibody light chains can be classified into κ and λ light chains. Heavy chains can be classified as µ, δ, γ, α, or ε, and define the antibody's isotype as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant region of antibody may mediate the binding of immunoglobulin to host tissues or factors, including various cells (e.g., effector cells) of immune system and first component (Clq) of classical complement system. The VH and VL regions can also be subdivided into highly variable regions called complementarity determining regions (CDRs), interspersed therewith more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3 and FR4. The variable regions (VH and VL) of each heavy chain/light chain pair respectively form an antibody binding site. The assignment of amino acids to regions or domains follows the definition of Bethesda M.d., Kabat Sequences of Proteins of Immunological Interest (National Institutes of Health, (1987 and 1991)), or Chothia & Lesk J. Mol. Biol. 1987; 196: 901-917; Chothia et al, Nature, 1989; 342: 878-883; or the IMGT numbering system, see, the definition of Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1): of D301-D307. The term "antibody" is not limited to any particular method of producing antibody, which includes, for example, recombinant antibodies, monoclonal antibodies, and polyclonal antibodies. Antibodies may be of different isotypes, for example, IgG (e.g., IgGl, IgG2, IgG3 or IgG4 subtype), IgA1, IgA2, IgD, IgE or IgM antibody.

As used herein, the term "antigen-binding fragment", also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989), which is incorporated herein by reference in its entirety for all purposes. The antigen-binding fragment of the antibody can be produced by recombinant DNA technology or by enzymatic or chemical cleavage of an intact antibody. In some cases, the antigen-binding fragment includes Fab, Fab', F(ab')2, Fd, Fv, dAb, and complementarity determining region (CDR) fragments, single chain antibody fragments (e.g., scFv), chimeric antibodies, diabodies, and polypeptides comprising at least a portion of the antibody sufficient to impart specific antigen binding ability to them.

As used herein, the term "Fd fragment" refers to an antibody fragment consisting of V_{H} and C_{H}1 domains; the term "Fv fragment" refers to an antibody fragment consisting of the V_{L} and VH domains of a single arm of an antibody; the term "dAb fragment" refers to an antibody fragment consisting of a VH domain (Ward et al., Nature 341:544-546 (1989)); the term "Fab fragment" refers to an antibody fragment consisting of V_{L}, V_{H}, C_{L} and CH1 domains; and the term "F(ab')2 fragment" refers to an antibody fragment comprising two Fab fragments linked by the disulfide bridge on a hinge region.

In some cases, the antigen-binding fragment of the antibody is a single chain antibody (e.g., scFv) in which the V_{L} and V_{H} domains are paired to form a monovalent molecule via a linker that enables them to produce a single polypeptide chain (see, e.g., Bird et al., Science 242:423-426 (1988) and Huston et al., Proc. Natl. Acad. Sci. USA 85:5879-5883 (1988)). Such scFv molecules may have a general structure: NH₂-V_{L}-linker-V_{H}-COOH or NH₂-V_{H}-linker-V_{L}-COOH. An appropriate linker in prior art consists of GGGGS amino acid sequence repeats or a variant thereof. For example, a linker having the amino acid sequence (GGGGS)₄ can be used, but variants thereof can also be used (Holliger et al. (1993), Proc. Natl. Acad. Sci. USA 90: 6444-6448). Other linkers that can be used in the present invention are described by Alfthan et al. (1995), Protein Eng. 8:725-731; Choi et al. (2001), Eur. J. Immunol. 31: 94-106; Hu et al. (1996), Cancer Res. 56:3055-3061; Kipriyanov et al. (1999), J. Mol. Biol. 293:41-56; and Roovers et al. (2001), Cancer Immunol.

In some cases, the antigen-binding fragment of the antibody is a diabody, that is, a bivalent antibody, in which the V_{H} and V_{L} domains are expressed on a single polypeptide chain. However, the linker used is too short to allow the pairing of the two domains on one chain, thereby the domains are forced to pair with the complementary domains on another chain and two antigen-binding sites are generated (see, e.g., Holliger P. et al., Proc. Natl. Acad. Sci. USA 90:6444-6448 (1993), and Poljak R. J. et al., Structure 2: 1121-1123 (1994)).

In other cases, the antigen-binding fragment of the antibody is a "bispecific antibody", which refers to a conjugate formed from a primary antibody (fragment) and a secondary antibody (fragment) or antibody analog via a linker; the methods of conjugation include, but are not limited to, chemical reaction, gene fusion, and enzyme catalysis. The antigen-binding fragment of the antibody may be a "multispecific antibody" including, for example, a trispecific antibody and a tetraspecific antibody, the former being an antibody with three different kinds of antigen-binding specificity, and the latter being an antibody with four different kinds of antigen-binding specificity. For example, a designed ankyrin repeat protein (DARPin) is linked to an IgG antibody, a scFv-Fc antibody fragment or combinations thereof, such as CN104341529A. An anti-IL-17a fynomer binds to an anti-IL-6R antibody, such as WO2015141862A1.

Antigen-binding fragments of antibodies (e.g., the antibody fragments described above) can be obtained from a given antibody (e.g., monoclonal antibody ADI-56127, ADI-56204 ADI-56249 or ADI-56145 provided in the present invention) using conventional techniques known to those skilled in the art (e.g., recombinant DNA technology or enzymatic or chemical cleavage), and the antigen-binding fragments of antibodies are screened for specificity in the same manner as for intact antibodies.

The terms "mcAb" and "monoclonal antibody" refer to an antibody or a fragment of antibody from a group of highly homologous antibody molecules, that is, a group of identical antibody molecules except for natural mutations that may occur spontaneously. Monoclonal antibody is highly specific for a single epitope on an antigen. Polyclonal antibody is relative to monoclonal antibody, which usually comprises at least two or more different antibodies, and these different antibodies usually recognize different epitopes on an antigen. Monoclonal antibodies can usually be obtained using the hybridoma technology that was first reported by Kohler et al. (Köhler G, Milstein C. Continuous cultures of fused cells secreting antibody of predefined specificity[J]. nature, 1975; 256(5517): 495), but also can be obtained using the recombinant DNA technology (e.g., see, U.S. Patent 4,816,567).

As used herein, the term "humanized antibody" refers to an antibody or antibody fragment obtained when all or a part of CDRs of a human immunoglobulin (receptor antibody) are replaced by the CDRs of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522-525; Reichmann et al., Nature, 1988; 332:323-329; Presta, Curr. Op. Struct. Biol. 1992; 2:593-596; and Clark M. Antibody humanization: a case of the 'Emperor's new clothes'? [J]. Immunol. Today, 2000; 21:397-402.

As used herein, the term "isolated" or "being isolated" refers to being obtained from the natural state by artificial means. If an "isolated" substance or ingredient occurs in nature, it may be due to a change in its natural environment, or the substance has been separated from its natural environment, or both. For example, a certain unisolated polynucleotide or polypeptide naturally exists in a living animal, and the same polynucleotide or polypeptide with high purity isolated from this natural state is called "isolated". The term "isolated" or "being isolated" does not exclude the admixture of artificial or synthetic substances, nor does it exclude the presence of other impure substances that do not affect the activity of the substance.

As used herein, the term "vector" refers to a nucleic acid delivery vehicle into which a polynucleotide can be inserted. When a vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage, and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may comprise a variety of expression-controlling elements, including, but not limited to, promoter sequence, transcription initiation sequence, enhancer sequence, selection element, and reporter gene. In addition, the vector may also comprise an origin of replication site.

As used herein, the term "host cell" refers to a cell that can be used to introduce a vector, which includes, but is not limited to, prokaryotic cell such as *Escherichia coli* or *Bacillus subtilis,* fungal cell such as yeast cell or *Aspergillus*, etc., insect cell such as *S2 Drosophila* cell or Sf9, or animal cell such as fibroblast, CHO cell, COS cell, NSO cell, HeLa cell, GS cell, BHK cell, HEK 293 cell or human cell.

As used herein, the terms "monoclonal antibody" and "McAb" have the same meaning and are used interchangeably; the terms "polyclonal antibody" and "PcAb" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable excipient" refers to a carrier and/or excipient that is pharmacologically and/or physiologically compatible with a subject and an active ingredient. It is well known in the art (see, for example, Remington's Pharmaceutical Sciences. Edited by Gennaro AR, 19th ed. Pennsylvania: Mack Publishing Company, 1995), and includes, but is not limited to: pH adjuster, surfactant, adjuvant, ionic strength enhancer. For example, the pH adjuster includes but is not limited to phosphate buffer; the surfactant includes but is not limited to cationic, anionic or nonionic surfactant such as Tween-80; and the ionic strength enhancer includes but is not limited to sodium chloride.

As used herein, the term "effective amount" refers to an amount sufficient to obtain, at least partially, the desired effect. For example, a prophylactically effective amount is an amount that is sufficient to prevent, arrest, or delay the occurrence of a disease (e.g., a tumor); a therapeutically effective amount is an amount that is sufficient to cure or at least partially prevent a disease and its complications in a patient who already has the disease.

As used herein, the terms "hybridoma" and "hybridoma cell line" are interchangeable, and when referring to "hybridoma" and "hybridoma cell line," it also includes subclones and progeny cells of the hybridoma.

In the present invention, unless otherwise specified, the terms "first" (e.g., first product) and "second" (e.g., second product) and the terms "third" (e.g., thihrd product) are used for distinction or clarity of expression and do not imply any typical order of precedence.

### Beneficial effects of the present invention

The present invention achieves one or more of the following technical effects as described in items (1) to (4):
(1) The antibody of the present invention is capable of binding to PVRIG with high affinity.
(2) The antibody of the present invention is likely to have a synergistic effect with anti-TIGIT antibodies or anti-PD-1 antibodies or anti-PD-L1 antibodies.
(3) The antibody of the present invention is effective in treating and/or preventing tumors, such as colorectal cancer, melanoma, lung cancer, kidney cancer, endometrial cancer, breast cancer, skin cancer, ovarian cancer, stomach cancer, head and neck cancer, liver cancer, brain tumor, urothelial cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer, among others.
(4) The antibody of the present invention has a low level of toxicity and side effects.

### Brief Description of the Drawings

Figs. 1A to 1B show the binding curve of the anti-PVRIG antibody to human PVRIG overexpressed on CHO cells.
Figs. 2A to 2B show the binding curve of the anti-PVRIG antibody to cynomolgus monkey PVRIG overexpressed on CHO cells.
Figs. 3A to 3B show the binding curve of the anti-PVRIG antibody to mouse PVRIG overexpressed on CHO cells.
Figs. 4A to 4B show the curve of the anti-PVRIG antibody blocking the binding of human PVRIG to human CD112 overexpressed on CHO cells.
Figs. 5A to 5B show the curve of the anti-PVRIG antibody blocking the binding of mouse CD112 to mouse PVRIG protein.
Fig. 6 shows the binding curve of the anti-PVRIG antibody to PVRIG on activated human primary T cells.
Fig. 7 shows the curve of anti-PVRIG antibody in combination with anti-TIGIT antibody blocking PVRIG/CD112 and TIGIT/CD155 signaling pathways.
Fig. 8 shows the curve of anti-PVRIG antibody in combination with anti-TIGIT and anti-PD-L1 antibodies blocking PVRIG/CD112, TIGIT/CD155 and PD-1/PD-L1 signaling pathways.
Figs. 9A to 9C show the results of ADCC assay on CHO cells expressing human PVRIG, cynomolgus monkey PVRIG and mouse PVRIG sequentially mediated by anti-PVRIG antibody.
Fig. 10 shows the efficacy of the anti-PVRIG antibody in the CT-26 tumor-bearing wild-type mouse model.
Fig. 11 shows the efficacy of the anti-PVRIG antibody in the NDG mouse model inoculated with mixed A375 and human PBMC.
Fig. 12 shows the efficacy of the anti-PVRIG antibody in combination with anti-TIGIT antibody in the NDG mouse model inoculated with mixed A375 and human PBMC.
Fig. 13 shows the efficacy of the anti-PVRIG antibody in combination with anti-TIGIT and anti-PD-1 antibodies in the NDG mouse model inoculated with mixed A375 and human PBMC.
Fig. 14 shows the in vivo half-life curve of the anti-PVRIG antibody in mice.

### Specific Models for Carrying Out the present invention

Embodiments of the present invention will now be described in detail with reference to the following examples, but it will be appreciated by those skilled in the art that the examples describe the present invention by way of example and are not intended to limit the scope of the present invention as claimed. Where specific conditions are not indicated in the examples, conventional conditions or conditions recommended by the manufacturer have been followed. Where the manufacturer is not indicated, the reagents or instruments used are conventional commercially available products.

Antibody Atezolizumab (ATE): anti-PD-L1 mono-antibody, trade name Tecentriq, Roche.

The following control antibodies used in the examples were expressed and purified from HEK293 cells:
COM701 is an anti-human PVRIG antibody from Compugen expressed by HEK293 cells with a light chain variable region sequence and a heavy chain variable region sequence corresponding to SEQ ID NO: 1372, SEQ ID NO: 1380 in US Patent US10227408B2.
Mab46 is an anti-human PVRIG antibody from Surface Oncology expressed by HEK293 cells with light chain variable region and heavy chain variable region sequences corresponding to SEQ ID NO: 912, SEQ ID NO: 918 in US Patent Publication US20200040081A1.
Tiragolumab is an anti-human TIGIT antibody from Genentech expressed by HEK293 cells with light and heavy chain sequences derived from WHO Drug Information, Vol. 31, No. 2, 2017, Proposed INN. List 117, CAS No. 1918185-84-8.

### Example 1: Sequence design, expression and purification of antibody

### 1.1 Sequence design of antibodies

Based on the yeast display antibody library (Adimab), amplification and optimization were performed according to the existing methods (see patent applications WO2009036379, WO2010105256 and WO2012009568). Yeast cells obtained by screening were induced to secrete and produce anti-PVRIG antibody (full-length IgG) by shaking at 30°C for 48 hours. After the induced expression was completed, the yeast cells were removed by centrifugation at 1300 rpm for 10 minutes, and the supernatant was collected. Protein A was used to purify the anti-PVRIG antibody in the supernatant, elution was performed with pH 2.0 acetic acid solution, and the anti-PVRIG antibodies ADI-56127, ADI-56204, ADI-56249 and ADI-56145 were harvested. Meanwhile, the antibodies ADI-56127, ADI-56204, ADI-56249, and ADI-56145 were digested with papain and the corresponding antibody Fab fragments were purified using Kappa Select (GE Healthcare).

The variable region sequences of the four antibodies ADI-56127, ADI-56204, ADI-56249, and ADI-56145 are shown below.

Heavy chain variable region sequence of ADI-56127:

QVQLVQSGAEVKKPGSSVKVSCKASGGTESVDAISWVRQAPGQGLEWMGDIIPFFDTDY
AQKFQGRVTITADESTSTAYMELSSLRSEDTAVYYCAREGGTSWTHFFDLWGRGTLVTV
SS (SEQ ID NO: 1)

Light chain variable region sequence of ADI-56127:

EIVLTQSPGTLSLSPGERATLSCRASQSVSSSYLAWYQQKPGQAPRLLIYGASSRATGIPD
RFSGSGSGTDFTLTISRLEPEDFAVYYCQQYGSSPITFGGGTKVEIK (SEQ ID NO: 2)

Heavy chain variable region sequence of ADI-56204:

QVQLVQSGAEVKKPGASVKVSCKASGYTFTHYGIYWVRQAPGQGLEWMGHIYAYSGD
TDYAQKLQGRVTMTTDTSTSTAYMELRSLRSDDTAVYYCARGSPTYEHFNWFDPWGQ
GTLVTVSS (SEQ ID NO: 3)

Light chain variable region sequence of ADI-56204:

DIQMTQSPSSLSASVGDRVTITCQASDDISDYLNWYQQKPGKAPKLLIYDASSLHTGVPS
RFSGSGSGTDFTFTISSLQPEDIATYYCQQYGNLPITFGGGTKVEIK (SEQ ID NO: 4)

Heavy chain variable region sequence of ADI-56249:

QVQLVQSGAEVKKPGSSVKVSCKASGGTFSTFAISWVRQAPGQGLEWMGDIIPIFGSNY
AQKFQGRVTITADESTSTAYMELSSLRSEDTAVYYCARGPLEGKVSPFDIWGQGTMVTV
SS (SEQ ID NO: 5)

Light chain variable region sequence of ADI-56249:

DIVMTQSPLSLPVTPGEPASISCRSSQSLLHSTGYNYLDWYLQKPGQSPQLLIYVGSTRAS
GVPDRFSGSGSGTDFTLKISRVEAEDVGVYYCMQERQFPITFGGGTKVEIK (SEQ ID NO: 6)

Heavy chain variable region sequence of ADI-56145:

QVQLVQSGAEVKKPGASVKVSCKASGYTFTSYGISWVRQAPGQGLEWVGWISSYDAST
KYSQELQGRVTMTTDTSTSTAYMELRSLRSEDTAVYYCARGSPIYEHFNWFDPWGQGT
LVTVSS (SEQ ID NO: 7)

Light chain variable region sequence of ADI-56145:

EIVLTQSPATLSLSPGERATLSCRASQSVSSYLAWYQQKPGQAPRLLIYDSSNRATGIPAR
FSGSGSGTDFTLTISSLEPEDFAVYYCQQLSHHPLTFGGGTKVEIK (SEQ ID NO: 8)

The CDRs for the above antibodies are listed below:
(1) ADI-56127

| | |
|---|---|
| HCDR1: | GTESVDAIS (SEQ ID NO: 9) |
| HCDR2: | DIIPFFDTDYAQKFQG (SEQ ID NO: 10) |
| HCDR3: | AREGGTSWTHFFDL (SEQ ID NO: 11) |
| LCDR1: | RASQSVSSSYLA (SEQ ID NO: 12) |
| LCDR2: | GASSRAT (SEQ ID NO: 13) |
| LCDR3: | QQYGSSPIT (SEQ ID NO: 14) |

(2) ADI-56204

| | |
|---|---|
| HCDR1: | YTFTHYGIY (SEQ ID NO: 15) |
| HCDR2: | HIYAYSGDTDYAQKLQG (SEQ ID NO: 16) |
| HCDR3: | ARGSPTYEHFNWFDP (SEQ ID NO: 17) |
| LCDR1: | QASDDISDYLN (SEQ ID NO: 18) |
| LCDR2: | DASSLHT (SEQ ID NO: 19) |
| LCDR3: | QQYGNLPIT (SEQ ID NO: 20) |

(3) ADI-56249

| | |
|---|---|
| HCDR1: | GTFSTFAIS (SEQ ID NO: 21) |
| HCDR2: | DIIPIFGSNYAQKFQG (SEQ ID NO: 22) |
| HCDR3: | ARGPLEGKVSPFDI (SEQ ID NO: 23) |
| LCDR1: | RSSQSLLHSTGYNYLD (SEQ ID NO: 24) |
| LCDR2: | VGSTRAS (SEQ ID NO: 25) |
| LCDR3: | MQERQFPIT (SEQ ID NO: 26) |

(4) ADI-56145

| | |
|---|---|
| HCDR1: | YTFTSYGIS (SEQ ID NO: 27) |
| HCDR2: | WISSYDASTKYSQELQG (SEQ ID NO: 28) |
| HCDR3: | ARGSPIYEHFNWFDP (SEQ ID NO: 29) |
| LCDR1: | RASQSVSSYLA (SEQ ID NO: 30) |
| LCDR2: | DSSNRAT (SEQ ID NO: 31) |
| LCDR3: | QQLSHHPLT (SEQ ID NO: 32) |

### 1.2 Expression and purification of antibodies and their variants

For antibodies ADI-56127, ADI-56204 and control antibody Mab46, their heavy chain variable region (VH) all were constructed into the wild-type human IgG1 heavy chain constant region (amino acid sequences as shown in SEQ ID NO: 33). In addition, for antibodies ADI-56127, ADI-56204, ADI-56249, ADI-56145 and control antibody Mab46, all of their heavy chain variable region (VH) were constructed into the heavy chain constant region modified by L234A, L235A (amino acid sequence as shown in SEQ ID NO: 34), respectively, constituting the complete "VH-CH1-CH2-CH3" antibody heavy chain. For the control antibody COM701, its heavy chain variable region was constructed into the human IgG4 heavy chain constant region (amino acid sequence as shown in SEQ ID NO: 35). In addition, the light chain variable regions (VL) of all the above antibodies were constructed into the human immunoglobulin kappa light chain constant region (amino acid sequence as shown in SEQ ID NO: 36). The antibody was transiently expressed and purified by the HEK293 expression system, and the specific operation was as follows: the pcDNA3.1 vector containing heavy and light chains of the antibody was transferred into HEK293 cells by chemical transfection method, which were cultured at 37°C, 8% CO₂ for 7 days. The cell fluid was collected and centrifuged at 13,000 rpm for 20 minutes. The supernatant was collected, purified with Protein A, and the antibody purity was detected by SEC, while the endotoxin content was controlled. The antibodies produced were named as ADI-56127-G1, ADI-56127-G1LALA, ADI-56204-G1, ADI-56204-G1LALA, ADI-56249-G1LALA, ADI-56145-G1LALA, Mab46-G1, Mab46-G1LALA and COM701-G4.

Wild-type human IgG1 heavy chain constant region:

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR

DELTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 33)

Heavy chain constant region modified by L234A, L235A:

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 34)

Human IgG4 heavy chain constant region:

ASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPSV
FLFPPKPKDTLMISRTPEVTCVVVDVSQEDPEVQFNWYVDGVEVHNAKTKPREEQFNST
YRVVSVLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVDKSRW
QEGNVFSCSVMHEALHNHYTQKSLSLSLG （SEQ ID NO: 35）

Human immunoglobulin kappa light chain constant region:


 (SEQ ID NO: 36)

### Example 2: Affinity detection

Biofilm layer optical interference technology (ForteBio) was used to determine the binding and dissociation rate constant (K_{D}) values of the antibodies and their variants or Fab fragments obtained in Example 1 to human, cynomolgus monkey and mouse PVRIG. ForteBio affinity measurement was performed according to existing methods (Este, P et al., High throughput solution-based measurement of antibody-antigen affinity and epitope binning. Mabs, 2013.5(2): p270-8). The amino acid sequences of the extracellular regions of human, cynomolgus monkey and mouse PVRIG were set forth in SEQ ID NO: 37-39, respectively.

The amino acid sequences of the extracellular region of human PVRIG:

TPEVWVQVRMEATELSSFTIRCGFLGSGSISLVTVSWGGPNGAGGTTLAVLHPERGIRQ
WAPARQARWETQSSISLILEGSGASSPCANTTFCCKFASFPEGSWEACGSLPPSSDPGLSA
PPTPAPILRAD (SEQ ID NO: 37)

The amino acid sequences of the extracellular region of cynomolgus monkey PVRIG:

TPEVWVQVQMEATELSSFTVHCGFLGPGSISLVTVSWGGPDGAGGTKLAVLHPELGTRQ
WAPARQARWETQSSISLALEDSGASSPFANTTFCCKFASFPEGSWESCGSLPPSSDPGLSA
PPTPVPILRAD (SEQ ID NO: 38)

The amino acid sequences of the extracellular region of mouse PVRIG:

SPEVWVQVQMEATNLSSFSVHCGVLGYSLISLVTVSCEGFVDAGRTKLAVLHPEFGTQQ
WAPARQAHWETPNSVSVTLTMGQSKARSSLANTTFCCEFVTFPHGSRVACRDLHRSDP
GLSAPTPALNLQAD (SEQ ID NO: 39)

Measurement of the monovalent affinity of intact antibodies (full-length IgG obtained from Adimab) to human PVRIG-his proteins: the sensor was equilibrated offline in an analysis buffer for 20 minutes, followed by online detection for 120 s to establish a baseline, and the intact PVRIG antibodies were loaded onto the AHQ sensor to reach a thickness of 1 nm for affinity detection. The antibody-loaded sensor was incubated in 100 nM PVRIG-his antigen to the plateau phase, and then the sensor was transferred to an analysis buffer and incubated for at least 2 minutes for measuring dissociation rate. The kinetic analysis was performed using a 1:1 binding model.

Measurement of the monovalent affinity of the Fab fragments of candidate antibodies (Fab fragments obtained from full-length IgG treated with papain) to human, cynomolgus monkey and mouse PVRIG-Fc proteins: the sensor was equilibrated offline in an analysis buffer for 20 minutes, followed by online detection for 120 s to establish a baseline, and the PVRIG-Fc antigens were loaded onto the AHQ sensor to reach a thickness of 1 nm for affinity detection. The antigen-loaded sensor was incubated in 100 nM Fab solution to the plateau phase, and then the sensor was transferred to an analysis buffer and incubated for at least 2 minutes for measuring dissociation rate. The kinetic analysis was performed using a 1:1 binding model.

Measurement of the bivalent affinity of intact antibodies to cynomolgus monkey and mouse PVRIG-Fc proteins: the sensor was equilibrated offline in an analysis buffer for 20 minutes, followed by online detection for 120 s to establish a baseline, and the intact PVRIG antibodies were loaded onto the AHQ sensor to reach a thickness of 1 nm for affinity detection. The antibody-loaded sensor was continued to be loaded in a high concentration of irrelevant intact antibody for 10 min, saturating the Fc-binding site on the AHQ sensor. The saturated sensor was incubated in 100 nM PVRIG-Fc antigens to the plateau phase, and then the sensor was transferred to an analysis buffer and incubated for at least 2 minutes for measuring dissociation rate. The kinetic analysis was performed using a 1:1 binding model.

In the above determination method, the K_{D} values of the candidate antibodies and their variants were shown in Table 1:

**Table 1: K_{D} values of anti-PVRIG candidates and their variants**

| Antibody | Monovalent affinity of IgG to human PVRIG protein | Monovalent affinity of Fab to human PVRIG-Fc protein | Bivalent affinity of IgG to cynomolgus PVRIG-Fc protein | Monovalent affinity of Fab to cynomolgus PVRIG-Fc protein | Bivalent affinity of IgG to mouse PVRIG-Fc protein | Monovalent affinity of Fab to mouse PVRIG-Fc protein |
|---|---|---|---|---|---|---|
| ADI-56127 | 3.64E-10 | 6.10E-11 | 1.81E-10 | 1.28E-10 | 1.66E-09 | 1.14E-07 |
| ADI-56204 | 4.25E-10 | 3.83E-10 | 1.97E-10 | 1.48E-08 | N.B. | NA |
| ADI-56249 | 8.72E-10 | 2.63E-10 | 2.44E-10 | 2.12E-08 | 2.62E-09 | NA |
| ADI-56145 | 2.04E-09 | 7.58E-10 | 6.23E-10 | 1.57E-07 | N.B. | NA |

| | | | | | | |
|---|---|---|---|---|---|---|
| Notes: "N.B.": no binding; "NA": not available. | | | | | | |

As seen from the results in Table 1: ADI-56127 and ADI-56249 have cross-binding activity with mouse PVRIG.

### Example 3: Binding and blocking activities of the anti-PVRIG antibodies to CHO cells overexpressing human/cynomolgus/mouse PVRIG

### 3.1 Detection of the binding activities of anti-PVRIG antibodies and their variants to human/cynomolgus/mouse PVRIG overexpressed on CHO cells based on flow cytometry

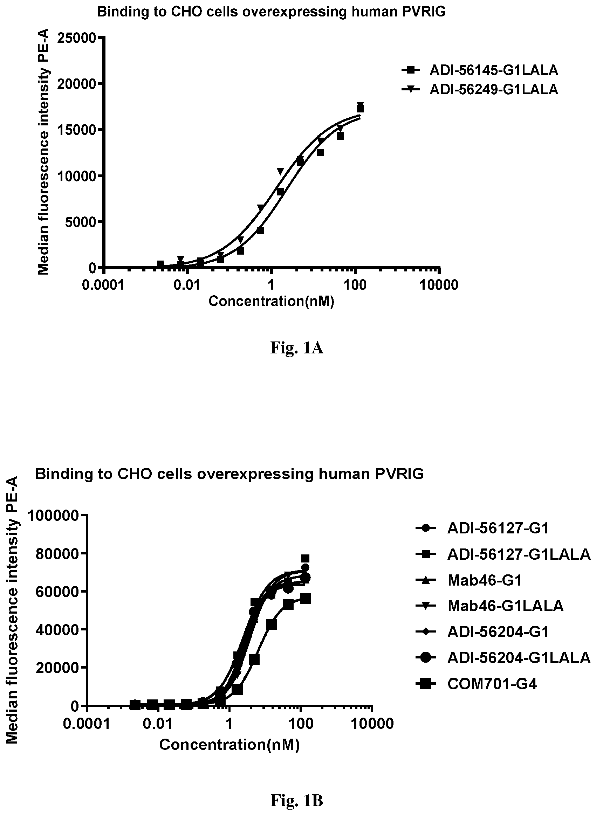

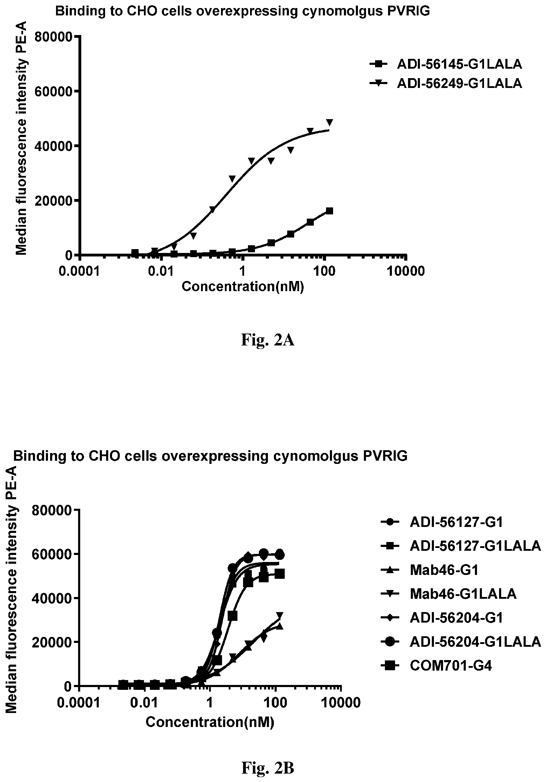

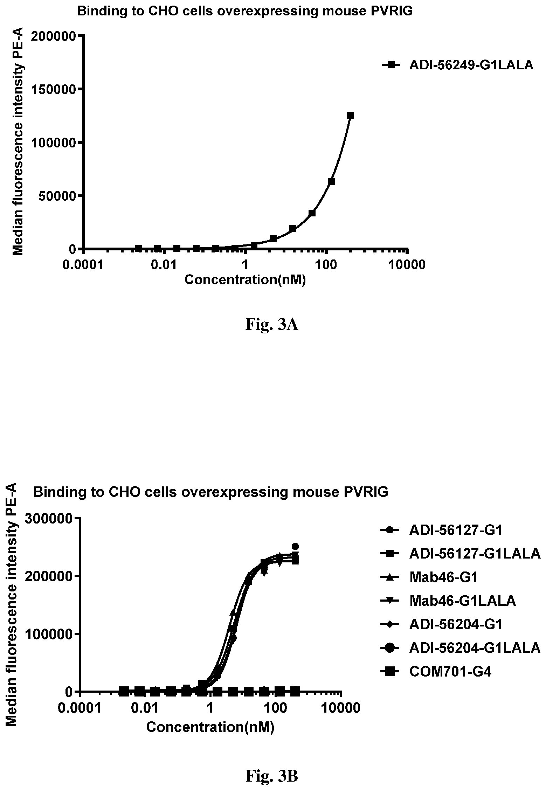

Specifically, CHO cells overexpressing human PVRIG (CHO-huPVRIG cells), CHO cells overexpressing cynomolgus PVRIG (CHO-cynoPVRIG cells) and CHO cells overexpressing mouse PVRIG (CHO-muPVRIG cells) were generated by transfection of the pCHO1.0 vector (purchased from Invitrogen) with cDNA encoding human PVRIG, cynomolgus PVRIG and mouse PVRIG cloned into the multiple cloning site (MCS), respectively, followed by pressure screening. The overexpressing cells were expanded and adjusted to an appropriate cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. Cells were resuspended with PBS, and detected on a CytoFlex flow cytometer, and the corresponding MFI was calculated. Graphpad software was used for graphical analysis to obtain EC₅₀ values. The results were shown in Table 2, Figs. 1A to 1B, Figs. 2A to 2B and Figs. 3A to 3B.

### 3.2 Detection of the blocking activities of anti-PVRIG antibodies and their variants in the binding of human PVRIG to human CD112 overexpressed on CHO cells based on flow cytometry

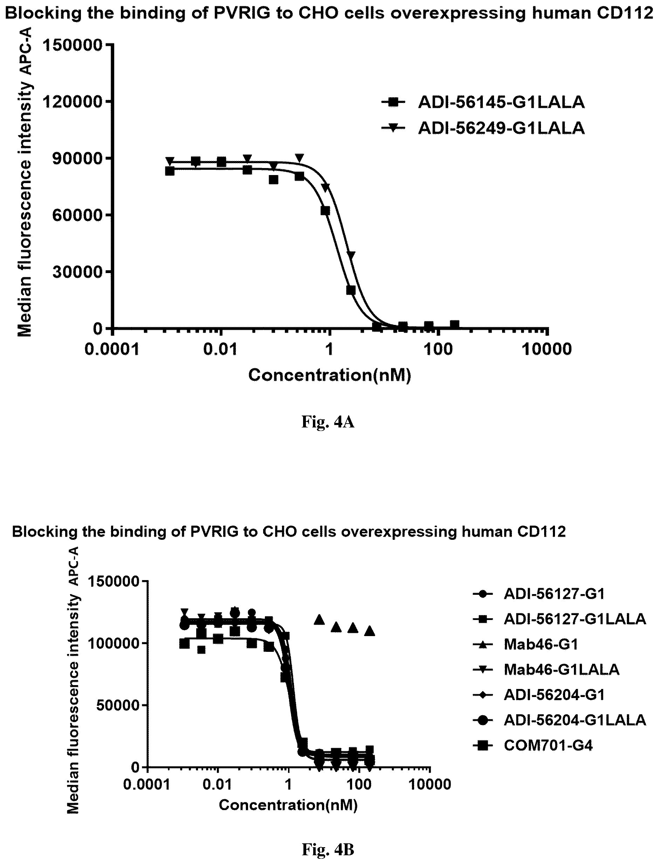

Specifically, CHO cells overexpressing human CD112 (CHO-huCD112 cells) were generated by transfection of the pCHO1.0 vector (purchased from Invitrogen) with cDNA encoding human CD112 cloned into the multiple cloning site (MCS), followed by pressure screening. The CHO-huCD112 cells were expanded and adjusted to a cell density of 2×10⁶ cells/mL, added to a 96-well flow plate at 100 µL/well, and centrifuged for later use. The purified monoclonal antibody was gradiently diluted with PBS and the diluted sample was added to a 96-well flow plate at 60 µL/well. Then, 1 µg/mL of human PVRIG protein with mouse IgG2a Fc tag was added at 60 µL/well, mixed and incubated at 4°C for 30 minutes. The above incubated samples were added at 100 µL/well to the 96-well flow plate containing CHO-huCD112 cells, incubated at 4°C for 30 min and washed twice with PBS. APC-conjugated goat anti-mouse IgG antibody diluted 100-fold with PBS was added at 100 µL/well, incubated at 4°C for 30 minutes and washed twice with PBS. Cells were resuspended with PBS at 100 µL/well, and detected on a CytoFlex flow cytometer, and the corresponding MFI was calculated. The results were shown in Table 2 and Figs. 4A to 4B.

### 3.3 Detection of the blocking activities of anti-PVRIG antibodies and their variants on the binding of mouse PVRIG protein to mouse CD112 protein based on the ELISA method

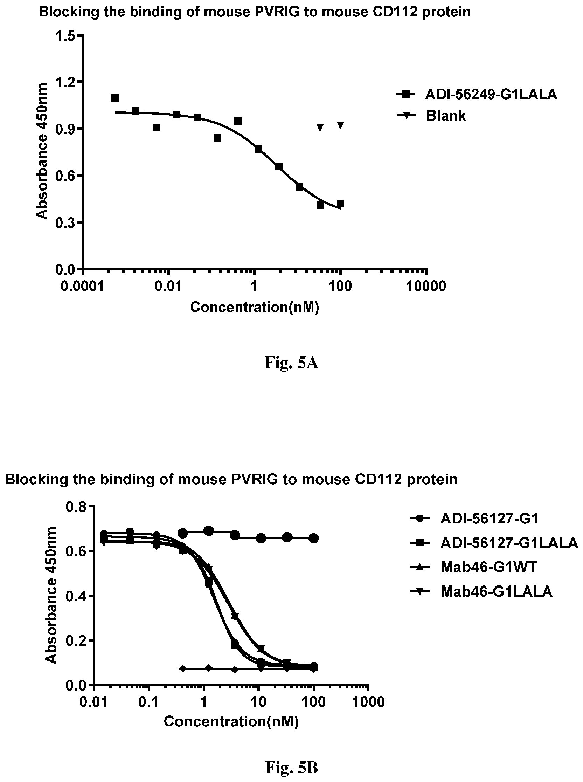

Specifically, mouse CD112-his protein diluted with 1 × coating buffer to a final concentration of 1 µg/mL was added to the ELISA plate at 100 µL/well, covered with the film, and coated overnight at 4°C. The coating solution in the ELISA plate was discarded, and the ELISA plate was washed three times with 1 × PBST and was blocked with 5% BSA/PBS at 200 µL/well for 2 hours at room temperature. During the blocking period, the samples to be tested were gradient diluted with 1% BSA/PBS to a final volume of 60 µL/well. Biotin-labeled mouse PVRIG huFc protein diluted to 2 µg/mL with 1% BSA/PBS was added to the above sample wells at 60 µL/well, mixed and incubated for 1 hour at room temperature. The blocking solution in the ELISA plate was discarded, and the above sample mixture was added at 100 µL/well and incubated for 2 hours at room temperature. The above co-incubated samples were discarded, the ELISA plate was washed three times with 1×PBST, and SA-HRP diluted with 1% BSA/PBS was added at 100 µL/well and incubated for 1 hour at room temperature. The SA-HRP dilution was discarded, the ELISA plate was washed three times with 1×PBST, ELISA Chromogen Solution was added at 100 µL/well and incubated for 1-3 minutes at room temperature, ELISA stopping solution was added at 50 µL/well and the absorbance values were read at 450 nm. Graphpad software was used to obtain the concentration-absorbance binding curves, the results were shown in Table 2, Figs. 5A to 5B.

**Table 2. Summary table of the binding activities and the blocking activities of anti-PVRIG antibodies and their variants on overexpressing cells**

| Antibody | EC50 of binding to CHO cells overexpressin g human PVRIG (nM) | EC50 of binding to CHO cells overexpressi ng cynomolgus PVRIG (nM) | EC50 of binding to CHO cells overexpressi ng mouse PVRIG (nM) | IC50 of blocking the binding of human PVRIG to CHO cells overexpressi ng human CD112 (nM) | IC50 of blocking the binding of mouse PVRIG to mouse CD112 protein (nM) |
|---|---|---|---|---|---|
| ADI-56249-G1LALA | 1.297 | 0.3959 | W.B. | 2.072 | 3.224 |
| ADI-56145-G1LALA | 2.285 | 37.61 | NA | 1.363 | NA |
| ADI-56204-G1 | 2.734 | 2.363 | N.B. | 1 | NA |
| ADI-56204-G1LALA | 2.503 | 1.967 | N.B. | 0.9906 | NA |
| ADI-56127-G1 | 2.514 | 1.955 | 6.469 | 1.229 | 1.568 |
| ADI-56127-G1LALA | 2.443 | 1.978 | 5.375 | 1.359 | 1.776 |
| Mab46-G1 | 2.999 | 8.18 | 3.877 | 1.153 | 2.834 |
| Mab46-G1LALA | 3.804 | 22.04 | 6.266 | 1.032 | 2.863 |
| COM701-G4 | 6.417 | 3.582 | N.B. | 1.097 | NA |

| | | | | | |
|---|---|---|---|---|---|
| Notes: "N.B.": no binding; and "NA": not available. | | | | | |

The following conclusions could be drawn from Table 2, Figs.1A to 1B, Figs 2A to 2B and Figs. 3A to 3B:
(1) The binding activities of ADI-56249-G1LALA, ADI-56145-G1LALA, ADI-56204-G1, ADI-56204-G1LALA, ADI-56127-G1 and ADI-56127-G1LALA to human PVRIG protein overexpressed on the surface of CHO cells were better than those of the control molecules Mab46-G1, Mab46-G1LALA and COM701-G4;
(2) The binding activities of ADI-56249-G1LALA, ADI-56204-G1, ADI-56204-G1LALA, ADI-56127-G1 and ADI-56127-G1LALA to cynomolgus PVRIG protein overexpressed on the surface of CHO cells were better than those of the control molecules Mab46-G1, Mab46-G1LALA and COM701-G4;
(3) ADI-56127-G1 and ADI-56127-G1LALA showed significant binding to mouse PVRIG protein overexpressed on the surface of CHO cells, and the binding activities is comparable to those of the control molecules Mab46-G1 and Mab46-G1LALA.

The following conclusions could be drawn from Table 2, Figs. 4A to 4B and Figs. 5A to 5B:
(1) The activities of ADI-56145-G1LALA, ADI-56204-G1, ADI-56204-G1LALA, ADI-56127-G1 and ADI-56127-G1LALA on blocking the binding of human PVRIG to human CD112 protein overexpressed on the surface of CHO cells were comparable to those of the of the control molecules Mab46-G1, Mab46-G1LALA and COM701-G4;
(2) Some of the anti-PVRIG candidates (ADI-56127-G1 and ADI-56127-G1LALA), which bind to mouse PVRIG protein overexpressed on the surface of CHO cells, were able to significantly block the binding of mouse PVRIG to mouse CD112 protein, and the blocking activities were superior to those of the control molecules Mab46-G1 and Mab46-G1LALA.

### Example 4: Binding of anti-PVRIG antibodies to PVRIG on the surface of primary T cells

The binding activity of the anti-PVRIG antibodies of the present invention to PVRIG on the surface of activated T cells was detected based on the flow cytometry detection method.

Specifically, human PBMC were sorted according to the experimental protocol provided by STEMCELL Company (stemcell, Cat. No.: #17951C) to obtain human total T cells. The T cells were adjusted to a concentration of 1.0×10⁶ cells/mL using X-VIVO15 medium (purchased from lonza, Cat. No.: 04-418Q), supplemented with 1µL of IL-2 stock solution (1,000,000 IU), and simultaneously added with CD3/CD28 Dynabeads (purchased from gibco, Cat. No.: 11132D) at a 1:1 ratio (bead-to-cell) and cultured in a 37°C, 5% CO₂ incubator for 48 hours. The activated T cells were adjusted to an appropriate cell density and added to a 96-well flow cytometry plate. After centrifugation, the gradiently diluted sample to be tested was added, and incubated at 4°C for 30 minutes. Washing was carried out twice with PBS, and a fluorescent secondary antibody diluted to an appropriate concentration was added, incubated at 4°C for 30 minutes, and washed twice with PBS. Cells were resuspended with PBS, and detected on a CytoFlex flow cytometer, and the corresponding MFI was calculated.

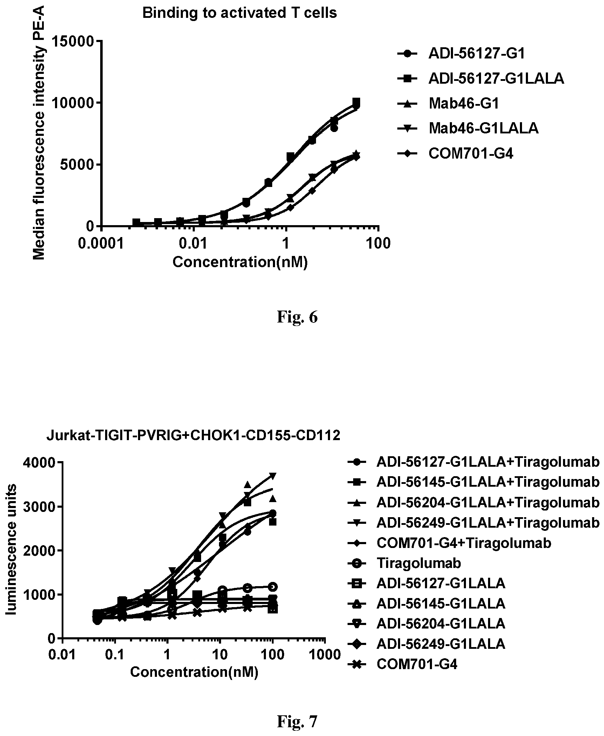

The results were shown in Fig. 6. The results showed that the anti-PVRIG antibodies ADI-56127-G1 and ADI-56127-G1LALA of the present invention could bind to PVRIG molecules on the surface of activated T cells, and the binding activities were better than those of the control molecules Mab46-G1, Mab46-G1LALA and COM701-G4.

### Example 5: Detection of the blocking effects of anti-PVRIG antibodies combined with anti-TIGIT antibody in the luciferase reporter system

To detect the combined blocking activity of anti-PVRIG antibody and anti-TIGIT antibodies at the cellular level, the following luciferase reporter system was constructed in this example. Briefly, a CHO-K1 cell line (CHO-K1-CD155-CD112) overexpressing human CD155, human CD112 and OKT-3 scFv and a Jurkat cell line (Jurkat-TIGIT-PVRIG-luc) overexpressing human TIGIT, human PVRIG and the companying NF-AT luciferase reporter gene were constructed by transfecting cells with lentivirals. This reporter system was subsequently used to perform relevant experiments.

Specifically, CHO-K1-CD155-CD112 functional cells were obtained by digestion, adjusted to the desired cell density, added at 100 µL/well to a 96-well white bottom plate, and cultured overnight for attachment. The next day, Jurkat-TIGIT-PVRIG-luc effector cell suspension was prepared, and the samples to be tested were gradiently diluted with reaction medium. The white bottom plate was taken out, the culture supernatant was discarded by pipetting, the above diluted samples were added to the white bottom plate at 40 µL/well, the Jurkat-TIGIT-PVRIG-luc effector cell suspension was added at 40 µL/well at the same time, and the incubation was performed in a 37°C, 5% CO₂ incubator for 6 hours. After incubation, the cells were added with Bio-Glo^{™} reagent at 80 µL/well, and a multifunctional microplate reader was used to read the luminescence signal values.

The results were shown in Fig. 7. The results showed that the anti-PVRIG antibodies ADI-56127-G1LALA, ADI-56145-G1LALA, ADI-56204-G1LALA and ADI-56249-G1LALA of the present invention in combination with the anti-TIGIT antibody Tiragolumab at a molar ratio of 1:1, respectively, were able to release the PVRIG inhibitory signal mediated by CD112 and the TIGIT inhibitory signal mediated by CD155, and enhanced the luciferase signal to a greater extent, and the activities were comparable to those of the control molecule COM701-G4.

### Example 6: Detection of the blocking effects of anti-PVRIG antibodies combined with anti-TIGIT and anti-PD-L1 antibodies in the luciferase reporter system

To detect the combined blocking activity of anti-PVRIG antibody with anti-TIGIT and anti-PD-L1 antibodies at the cellular level, the following luciferase reporter system was constructed in this example. Briefly, based on Example 5, lentivirus was used to infect CHO-K1-CD155-CD112 to overexpress PD-L1 to obtain CHO-K1-CD155-CD112-PD-L1 functional cells, and lentivirus was used to infect Jurkat-TIGIT-PVRIG-luc to overexpress PD-1 to obtain Jurkat-TIGIT-PVRIG-PD-1-luc effector cell suspension, and this reporter system was used in subsequent experiments.

Specifically, CHO-K1-CD155-CD112-PD-L1 functional cells were obtained by digestion, adjusted to the desired cell density, added at 100 µL/well to a 96-well white bottom plate, and cultured overnight for attachment. The next day, Jurkat-TIGIT-PVRIG-PD-1-luc effector cell suspension was prepared, and the samples to be tested were gradiently diluted with reaction medium. The white bottom plate was taken out, the culture supernatant was discarded by pipetting, the above diluted samples were added to the white bottom plate at 40 µL/well, the Jurkat-TIGIT-PVRIG-PD-1-luc effector cell suspension was added at 40 µL/well at the same time, and the incubation was performed in a 37°C, 5% CO₂ incubator for 6 hours. After incubation, the cells were added with Bio-Glo^{™} reagent at 80 µL/well, and a multifunctional microplate reader was used to read the luminescence signal values.

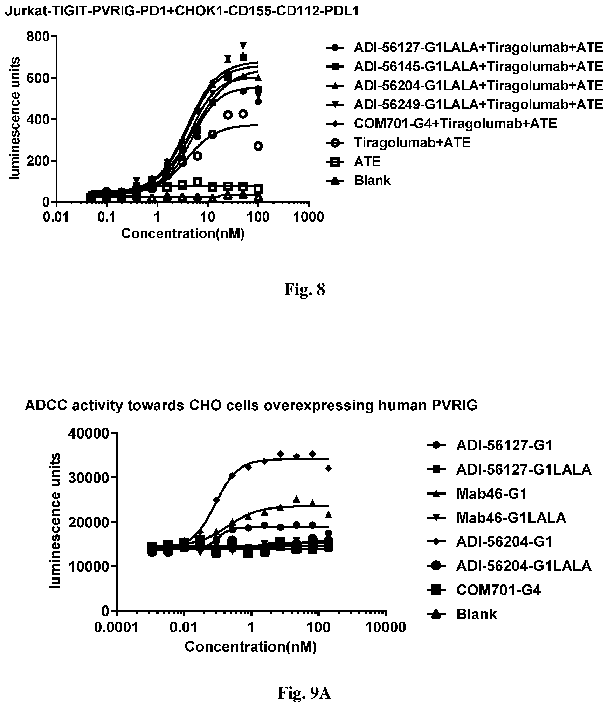

The results were shown in Fig. 8. The results showed that the anti-PVRIG antibodies ADI-56127-G1LALA, ADI-56145-G1LALA, ADI-56204-G1LALA and ADI-56249-G1LALA of the present invention combined with anti-TIGIT antibody Tiragolumab and anti-PD-L1 antibody Atezolizumab (ATE) at a molar ratio of 1:1:1, were able to further enhance the luciferase signal by blocking the inhibitory signal mediated by PVRIG/CD112, TIGIT/CD155, and PD-1/PD-L1, respectively, with activities comparable to those of the control molecule, COM701-G4.

### Example 7: Detection of in vitro ADCC activity of anti-PVRIG antibodies

The in vitro ADCC activities of the anti-PVRIG antibodies of the present invention were detected based on the luciferase reporter gene system.

Specifically, Jurkat-NFAT-Luciferase-CD16 ADCC effector cells (purchased from Promega) were expanded, and the cells were resuspended to 4×10⁶ cells/mL in RPMI1640 medium containing 10% low IgG FBS. CHO-huPVRIG cells, CHO-cynoPVRIG cells and CHO-muPVRIG cells were resuspended in RPMI1640 medium containing 10% low IgG FBS, and diluted to 1.6×10⁶ cells/mL. The above three cell suspensions were mixed with the Jurkat-NFAT-Luciferase-CD16 cells at a ratio of 1:1, respectively, and added at 50 µL/well into a sterile 96-well white bottom plate. The antibody samples to be tested which were gradiently diluted in RPMI1640 medium containing 10% low IgG FBS were added. Co-incubation was carried out at 37°C, 5% CO₂ for 6 hours. After incubation, the cells were taken out and equilibrated at room temperature for 5 minutes, added with Bio-Glo TM reagent at 100 µL/well, and a multifunctional microplate reader was used to read the luminescence signal values.

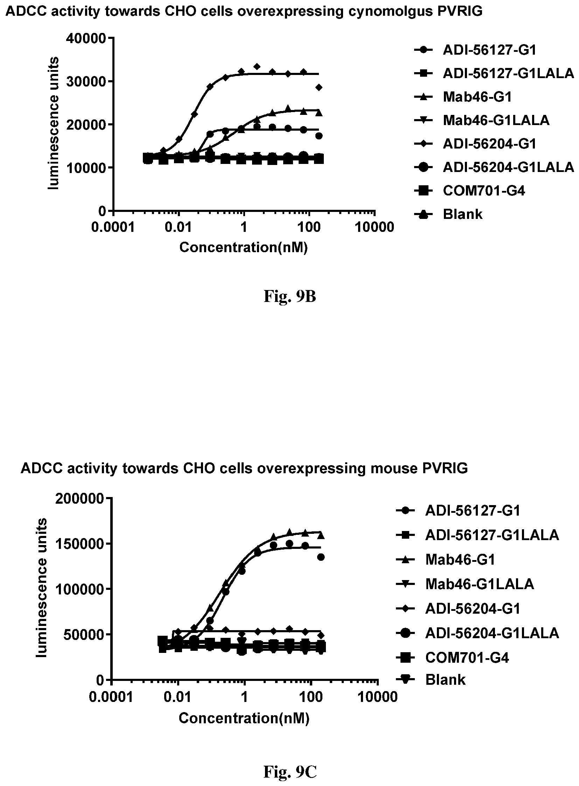

The results were shown in Figs. 9A to 9C. The results showed that the anti-PVRIG antibodies ADI-56204-G1 and ADI-56127-G1 of the present invention could mediate the in vitro ADCC activities on CHO-huPVRIG cells, CHO-cynoPVRIG cells and CHO-muPVRIG cells in the luciferase reporter system. ADI-56204-G1 mediated a stronger ADCC activity against CHO-huPVRIG cells and CHO-cynoPVRIG cells than that of the control molecule Mab46-G1, while the ADCC activity mediated by ADI-56127-G1 on CHO-huPVRIG cells, CHO-cynoPVRIG cells and CHO-muPVRIG cells was comparable to that of the control molecule Mab46-G1.

### Example 8: In vivo pharmacodynamic study of anti-PVRIG antibody in wild-type Balb/c mice

In this experiment, wild-type Balb/c mice were inoculated with CT-26 colon cancer cells (purchased from Gempharmatech Co., Ltd.) to determine the anti-tumor effect of the anti-PVRIG antibody of the present invention.

Specifically, a suspension of CT-26 cells was prepared, and approximately 5×10⁵ cells in 0.1 mL were injected subcutaneously into the right inguinal region of mice to establish a CT-26 tumor-bearing mouse model. Once the average tumor volume reached about 100 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 3.

**Table 3: Experimental protocol for tumor inhibitory activity of anti-PVRIG antibody**

| Group | Administration dose | Administration frequency |
|---|---|---|
| PBS | N/A | Q3d×4 |
| ADI-56127-G1 | 10 mg/kg | Q3d×4 |
| ADI-56127-G1LALA | 10 mg/kg | Q3d×4 |

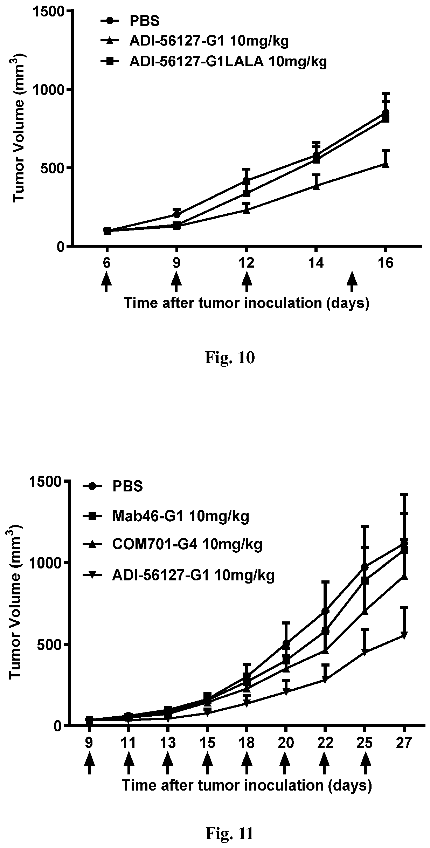

The results were shown in Fig. 10. The results showed that the anti-PVRIG antibody ADI-56127-G1 of the present invention could significantly inhibit the growth of CT26 tumors in mice.

### Example 9: In vivo pharmacodynamic study of anti-PVRIG antibody in NDG mice inoculated with mixed A375 and human PBMCs

In this experiment, an A375 huPBMC model was established in B-NDG mice inoculated with mixed A375 (purchased from Addexbio, Cat. No.: C0020004, a malignant human melanoma cell) and human PBMC cells (Milestone Biotechnologies, A10S033014/PB100C) to determine the anti-tumor effect of the anti-PVRIG antibody of the present invention. In this regard, a humanized tumor mouse model with a partially recombinant human immune system was generated by inoculating human immune cells (PBMC) into immunodeficient mice.

Specifically, A375 cells were first mixed 1:1 with human PBMCs to form a 0.1 mL cell suspension, and the A375 huPBMC model was established in the right groin of mice by subcutaneous injection. Once the average tumor volume reached about 30 to 50 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 4.

**Table 4: Dosage regimen of anti-PVRIG antibody in A375 tumor-PBMC model**

| Group | Administration dose | Administration frequency |
|---|---|---|
| PBS | N/A | Q2-3d×8 |
| ADI-56127-G1 | 10 mg/kg | Q2-3d×8 |
| Mab46-Gl | 10 mg/kg | Q2-3d×8 |
| COM701-G4 | 10 mg/kg | Q2-3d×8 |

The results were shown in Fig. 11. The results showed that the anti-PVRIG antibody ADI-56127-G1 of the present invention can significantly inhibit the growth of A375 tumors in mice, and the anti-tumor activity is superior to that of the control molecules Mab46-G1 and COM701-G4.

### Example 10: In vivo pharmacodynamic study of anti-PVRIG antibody combined with anti-TIGIT antibody in NDG mice inoculated with mixed A375 and human PBMCs

In this experiment, human PBMCs with A375 tumor-bearing mouse models were first established by subcutaneous mixed inoculation (the modeling procedure was the same as in Example 9). Once the average tumor volume reached about 60 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 5.

**Table5: Dosage regimen of anti-PVRIG antibody with anti-TIGIT antibody in A375 tumor-bearing model**

| Group | Administration dose | Administration frequency |
|---|---|---|
| Negative control | N/A | Q2d×6 |
| ADI-56127-G1 | 10 mg/kg | Q2d×6 |
| Anti-TIGIT-mAb | 10 mg/kg | Q2d×6 |
| ADI-56127-Gl+Anti-TIGIT-mAb | 10 mg/kg+10 mg/kg | Q2d×6 |

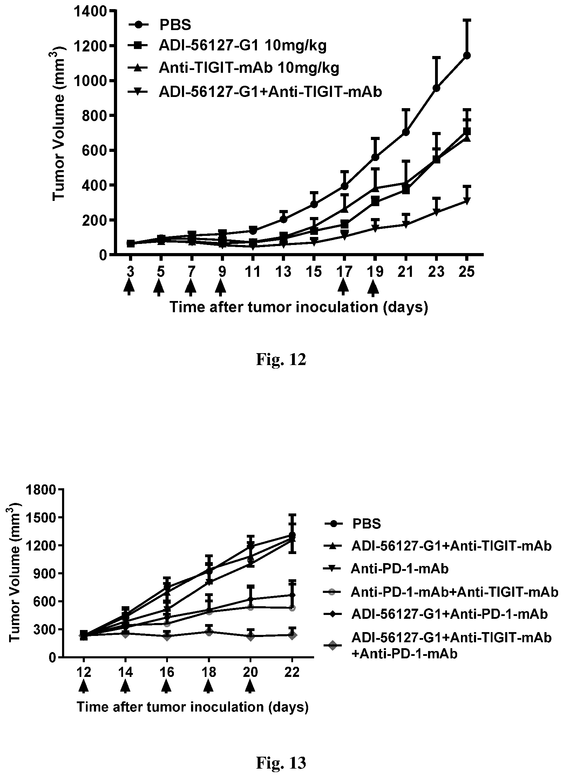

The results were shown in Fig. 12. The results showed that the combined administration of the anti-PVRIG antibody ADI-56127-G1 of the present invention with the anti-TIGIT monoclonal antibody has a more significant tumor suppressive effect than that of monotherapy with ADI-56127-G1 or Anti-TIGIT-mAb.

The sequences of anti-TIGIT monoclonal antibody used in this example are shown below:
Amino acid sequence of the VH of anti-TIGIT antibody: 
QLQLQESGPGLVKPAETLSLTCTVSGGSISSYDHYWTWIRQPPGKGLEWIGTVYYSGSTF
HNPSLKSRVTIPVDTSKNQFSLKLSSVTAADTAVYYCARVGPDVSHPPFDYWGQGTLVT
VSS (SEQ ID NO: 40)
Amino acid sequence of the VL of anti-TIGIT antibody: 
DIQMTQSPSSLSASVGDRVTITCRASQSISSYLNWYQQKPGKAPKLLIYAASSLQSGVPSR
FSGSGSGTDFTLTISSLQPEDFATYYCQQSYSTPITFGGGTKVEIK (SEQ ID NO: 41)
The CDRs for the above anti-TIGIT antibody are listed below:
   HCDR1: GSISSYDHYWT (SEQ ID NO: 42)
   HCDR2: TVYYSGSTFHNPSLKS (SEQ ID NO: 43)
   HCDR3: ARVGPDVSHPPFDY (SEQ ID NO: 44)
   LCDR1: RASQSISSYLN (SEQ ID NO: 45)
   LCDR2: AASSLQS (SEQ ID NO: 46)
   LCDR3: QQSYSTPIT (SEQ ID NO: 47)

The heavy chain constant region of anti-TIGIT monoclonal antibody used in this example was the wild-type human IgG1 heavy chain constant region (SEQ ID NO: 33).

The light chain constant region of anti-TIGIT monoclonal antibody used in this example was the human kappa light chain constant region (SEQ ID NO: 36).

### Example 11: In vivo pharmacodynamic study of anti-PVRIG antibody combined with anti-PD-1 and anti-TIGIT antibody in NDG mice inoculated with mixed A375 and human PBMCs

In this experiment, human PBMCs with A375 tumor-bearing mouse models were first established by subcutaneous mixed inoculation (the modeling procedure was the same as in Example 9). Once the average tumor volume reached about 200 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 6.

**Table 6: Dosage regimen of anti-PVRIG antibody with anti-PD-1 and anti-TIGIT antibody in A375 tumor-bearing model**

| Group | Administration dose | Administration frequency |
|---|---|---|
| PBS | N/A | Q2d×5 |
| ADI-56127-Gl+Anti-TIGIT-mAb | 10 mg/kg+10 mg/kg | Q2d×5 |
| Anti-PD-1-mAb | 5 mg/kg | Q2d×5 |
| Anti-PD-1-mAb+Anti-TIGIT-mAb | 5 mg/kg+10 mg/kg | Q2d×5 |
| Anti-PD-1-mAb+ADI-56127-Gl | 5 mg/kg+10 mg/kg | Q2d×5 |
| ADI-56127-G1+Anti-PD-1- mAb+Anti-TIGIT-mAb | 5 mg/kg+10 mg/kg+10 mg/kg | Q2d×5 |

As shown in Fig. 13, the results indicated that the combined administration of the anti-PVRIG antibody ADI-56127-G1 of the present invention, anti-TIGIT monoclonal antibody and anti-PD-1 antibody can effectively inhibit tumor growth, with significantly greater tumor suppressive activity against A375 compared to other treatment groups.

The sequences of the anti-TIGIT monoclonal antibody used in this example is consistent with Example 10.

The sequences of anti-PD-1 monoclonal antibody used in this example are shown below:
Amino acid sequence of the VH of anti-PD-1 antibody 
QVQLVQSGAEVKKPGASVKVSCKASGYTFTEYYIYWVRQAPGQGLEWIGGINPSNGGT
NFNEKFKPRVTMTVDTSTSTAYMELSSLRSEDTAVYYCTVRDFRFDKGFKYWGQGTLV
TVSS (SEQ ID NO: 48)
Amino acid sequence of the VL of anti-PD-1 antibody

EIVLTQSPATLSLSPGERATLSCRASKSVSTSGLNYVHWYQRKPGQAPRLLIYLGSYLDS
GVPARFSGSGSGTDFTLTISSLEPEDFAVYYCQQSWELPLTFGGGTKVEIK (SEQ ID NO:
49)

The 6 CDRs for the above anti-PD-1 antibody are listed below:
HCDR1: FTEYYIY (SEQ ID NO: 50)
HCDR2: NPSNGGTNFNEKFKP (SEQ ID NO: 51)
HCDR3: RDFRFDKGFKY (SEQ ID NO: 52)
LCDR1: SKSVSTSGLNYVH (SEQ ID NO: 53)
LCDR2: SYLDS (SEQ ID NO: 54)
LCDR3: SWELPLT (SEQ ID NO: 55)

The heavy chain constant region of anti-PD-1 monoclonal antibody used in this example was the IgG1 heavy chain constant region modified by L234A, L235A (SEQ ID NO: 34).

The light chain constant region of anti-PD-1 monoclonal antibody used in this example was the human kappa light chain constant region (SEQ ID NO: 36).

### Example 12: In vivo half-life study of the anti-PVRIG antibody in mice

The in vivo half-life of the anti-PVRIG antibody of the present invention, ADI-56127-G1, was detected in mice by the tail vein single injection method.

Specifically, Balb/c mice, half male and half female, were used in this experiment and were housed in a 12/12-hour light/dark regulated environment at a temperature of 24°C ± 2°C and a humidity of 40%-70%, with free access to water and food. On the day of the experiment, Balb/c mice received a single tail vein injection of monoclonal antibody molecules at a dose of 10 mg/kg. Blood collection time points: Blood was collected from the orbits of the mice at 5 minutes, 0.5 hours, 2 hours, 6 hours, 24 hours, 48 hours, 96 hours, 168 hours, 336 hours, and 504 hours after the administration. Whole blood samples were placed at 2°C to 8°C for 30 minutes and centrifuged at 12,000 rpm for 5 minutes to collect serum. The collected serum was centrifuged at 2° C to 8°C, 12,000 rpm for 5 minutes and stored at -80°C. The amount of the monoclonal antibody molecules in the serum was detected by ELISA.

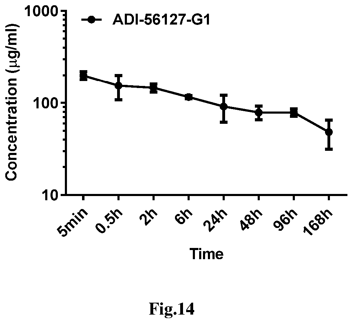

The results were shown in Fig. 14. The results showed that the in vivo half-life of the anti-PVRIG antibody ADI-56127-G1 of the present invention after a single injection into mice was 160 hours.

Although the specific embodiments of the present invention have been described in detail, those skilled in the art will understand that various modifications and changes can be made to the details based on all teachings that have been published, and these changes are within the protection scope of the present invention. The full scope of the present invention is given by the appended claims and any equivalents thereof.

## Claims

1. An anti-PVRIG antibody or an antigen-binding fragment thereof, wherein, the antibody comprises a heavy chain variable region comprising HCDR1 to HCDR3 and a light chain variable region comprising LCDR1 to LCDR3, wherein:
the heavy chain variable region of the antibody comprises,
HCDR1 with the amino acid sequence as set forth in SEQ ID NO:9, HCDR2 with the amino acid sequence as set forth in SEQ ID NO:10 and HCDR3 with the amino acid sequence as set forth in SEQ ID NO:11,
HCDR1 with the amino acid sequence as set forth in SEQ ID NO:15, HCDR2 with the amino acid sequence as set forth in SEQ ID NO:16 and HCDR3 with the amino acid sequence as set forth in SEQ ID NO:17,
HCDR1 with the amino acid sequence as set forth in SEQ ID NO:21, HCDR2 with the amino acid sequence as set forth in SEQ ID NO:22 and HCDR3 with the amino acid sequence as set forth in SEQ ID NO:23, or
HCDR1 with the amino acid sequence as set forth in SEQ ID NO:27, HCDR2 with the amino acid sequence as set forth in SEQ ID NO:28 and HCDR3 with the amino acid sequence as set forth in SEQ ID NO:29;
and
the light chain variable region of the antibody comprises,
LCDR1 with the amino acid sequence as set forth in SEQ ID NO:12, LCDR2 with the amino acid sequence as set forth in SEQ ID NO:13 and LCDR3 with the amino acid sequence as set forth in SEQ ID NO:14,
LCDR1 with the amino acid sequence as set forth in SEQ ID NO:18, LCDR2 with the amino acid sequence as set forth in SEQ ID NO:19 and LCDR3 with the amino acid sequence as set forth in SEQ ID NO:20,
LCDR1 with the amino acid sequence as set forth in SEQ ID NO:24, LCDR2 with the amino acid sequence as set forth in SEQ ID NO:25 and LCDR3 with the amino acid sequence as set forth in SEQ ID NO:26, or
LCDR1 with the amino acid sequence as set forth in SEQ ID NO:30, LCDR2 with the amino acid sequence as set forth in SEQ ID NO:31 and LCDR3 with the amino acid sequence as set forth in SEQ ID NO:32.

2. The anti-PVRIG antibody or antigen-binding fragment thereof according to claim 1, wherein,
the heavy chain variable region of the antibody comprises HCDR1 with the amino acid sequence as set forth in SEQ ID NO:9, HCDR2 with the amino acid sequence as set forth in SEQ ID NO:10 and HCDR3 with the amino acid sequence as set forth in SEQ ID NO:11, and, the light chain variable region of the antibody comprises LCDR1 with the amino acid sequence as set forth in SEQ ID NO: 12, LCDR2 with the amino acid sequence as set forth in SEQ ID NO: 13 and LCDR3 with the amino acid sequence as set forth in SEQ ID NO:14;
the heavy chain variable region of the antibody comprises HCDR1 with the amino acid sequence as set forth in SEQ ID NO:15, HCDR2 with the amino acid sequence as set forth in SEQ ID NO:16 and HCDR3 with the amino acid sequence as set forth in SEQ ID NO:17, and, the light chain variable region of the antibody comprises LCDR1 with the amino acid sequence as set forth in SEQ ID NO: 18, LCDR2 with the amino acid sequence as set forth in SEQ ID NO: 19 and LCDR3 with the amino acid sequence as set forth in SEQ ID NO:20;
the heavy chain variable region of the antibody comprises HCDR1 with the amino acid sequence as set forth in SEQ ID NO:21, HCDR2 with the amino acid sequence as set forth in SEQ ID NO:22 and HCDR3 with the amino acid sequence as set forth in SEQ ID NO:23, and, the light chain variable region of the antibody comprises LCDR1 with the amino acid sequence as set forth in SEQ ID NO:24, LCDR2 with the amino acid sequence as set forth in SEQ ID NO:25 and LCDR3 with the amino acid sequence as set forth in SEQ ID NO:26;
or
the heavy chain variable region of the antibody comprises HCDR1 with the amino acid sequence as set forth in SEQ ID NO:27, HCDR2 with the amino acid sequence as set forth in SEQ ID NO:28 and HCDR3 with the amino acid sequence as set forth in SEQ ID NO:29, and, the light chain variable region of the antibody comprises LCDR1 with the amino acid sequence as set forth in SEQ ID NO:30, LCDR2 with the amino acid sequence as set forth in SEQ ID NO:31 and LCDR3 with the amino acid sequence as set forth in SEQ ID NO:32.

3. The anti-PVRIG antibody or antigen-binding fragment thereof according to any one of claims 1 to 2, wherein, the heavy chain variable region has an amino acid sequence selected from SEQ ID NO: 1, SEQ ID NO: 3, SEQ ID NO: 5 and SEQ ID NO: 7;
and
the light chain variable region has an amino acid sequence selected from SEQ ID NO:2, SEQ ID NO:4, SEQ ID NO:6 and SEQ ID NO:8.

4. The anti-PVRIG antibody or antigen-binding fragment thereof according to any one of claims 1 to 3, wherein,
the heavy chain variable region has an amino acid sequence as set forth in SEQ ID NO: 1, and the light chain variable region has an amino acid sequence as set forth in SEQ ID NO: 2;
the heavy chain variable region has an amino acid sequence as set forth in SEQ ID NO:3, and the light chain variable region has an amino acid sequence as set forth in SEQ ID NO:4;
the heavy chain variable region has an amino acid sequence as set forth in SEQ ID NO:5, and the light chain variable region has an amino acid sequence as set forth in SEQ ID NO:6;
or
the heavy chain variable region has an amino acid sequence as set forth in SEQ ID NO:7, and the light chain variable region has an amino acid sequence as set forth in SEQ ID NO:8.

5. The anti-PVRIG antibody or antigen-binding fragment thereof according to any one of claims 1 to 4, wherein,
the heavy chain constant region of the antibody is Ig gamma-1 chain C region or Ig gamma-4 chain C region; the light chain constant region is Ig kappa chain C region;
preferably, the heavy chain constant region of the antibody has an amino acid sequence as set forth in SEQ ID NO: 33, SEQ ID NO: 34 or SEQ ID NO: 35, and the light chain constant region of the antibody has an amino acid sequence as set forth in SEQ ID NO: 36.

6. The anti-PVRIG antibody or antigen-binding fragment thereof according to any one of claims 1 to 5, wherein,
the heavy chain constant region of the antibody comprises a L234A mutation and a L235A mutation according to the EU numbering system.

7. The anti-PVRIG antibody or antigen-binding fragment thereof according to any one of claims 1 to 6, wherein, the anti-PVRIG antibody or antigen-binding fragment is selected from the group consisting of Fab, Fab', F(ab')₂, Fd, Fv, dAb, a complementarity determining region fragment, a single chain antibody, a humanized antibody, a chimeric antibody and a diabody.

8. The anti-TIGIT antibody or antigen-binding fragment thereof according to any one of claims 1 to 7, wherein,
the antibody comprises a non-CDR region, and the non-CDR region is derived from a species other than murine, such as from a human antibody.

9. The anti-PVRIG antibody or antigen-binding fragment thereof according to any one of claims 1 to 8, wherein,
the antibody binds to human PVRIG protein overexpressed on the surface of CHO cells with an EC₅₀ of less than 3nM, less than 2.9nM or less than 2.8nM; preferably, the EC₅₀ is measured by flow cytometry assay method.

10. An isolated nucleic acid molecule, which encodes the anti-TIGIT antibody or antigen-binding fragment thereof according to any one of claims 1 to 9.

11. A vector, which comprises the isolated nucleic acid molecule according to claim 10.

12. A host cell, which comprises the isolated nucleic acid molecule according to claim 10 or the vector according to claim 11.

13. A conjugate, which comprises an antibody and a conjugated moiety, wherein, the antibody is an anti-PVRIG antibody or an antigen-binding fragment thereof according to any one of claims 1 to 9, and the conjugated moiety is a detectable label; preferably, the conjugated moiety is a radioactive isotope, a fluorescent substances, colored substances or enzymes.

14. A kit, which comprises the anti-PVRIG antibody or antigen-binding fragment thereof according to any one of claims 1 to 9, or the conjugate according to claim 13;
preferably, the kit further comprises a second antibody that specifically recognizes the antibody; optionally, the second antibody further comprises a detectable label, such as a radioactive isotope, a fluorescent substance, a colored substance or an enzyme.

15. A pharmaceutical composition, which comprises the anti-PVRIG antibody or antigen-binding fragment thereof according to any one of claims 1 to 9, or the conjugate according to claim 13; optionally, the pharmaceutical composition further comprises one or more pharmaceutically acceptable excipients.

16. The pharmaceutical composition of claim 15, further comprising one or more anti-TIGIT antibodies;
preferably, the pharmaceutical composition further comprises one or more anti-PD-1 antibodies or one or more anti-PD-L1 antibodies.

17. The pharmaceutical composition according to any one of claims 15 to 16, wherein,
the molar ratio of anti-PVRIG antibody or its antigen-binding fragment to anti-TIGIT antibody is from (1:5) to (5:1);
and / or
the molar ratio of anti-PVRIG antibody or antigen-binding fragment to anti-PD-1 antibody or anti-PD-L1 antibody is from (1:5) to (5:1).

18. The pharmaceutical composition according to any one of claims 16 to 17, wherein, the anti-TIGIT antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-TIGIT antibody comprises HCDR1 with the amino acid sequence as set forth in SEQ ID NO:42, HCDR2 with the amino acid sequence as set forth in SEQ ID NO:43 and HCDR3 with the amino acid sequence as set forth in SEQ ID NO:44, and, the light chain variable region of the anti-TIGIT antibody comprises LCDR1 with the amino acid sequence as set forth in SEQ ID NO:45, LCDR2 with the amino acid sequence as set forth in SEQ ID NO:46 and LCDR3 with the amino acid sequence as set forth in SEQ ID NO:47;
preferably, the heavy chain variable region of the anti-TIGIT antibody has an amino acid sequence as set forth in SEQ ID NO:40, and the light chain variable region has an amino acid sequence as set forth in SEQ ID NO:41.

19. The pharmaceutical composition according to any one of claims 16 to 18, wherein, the anti-PD-1 antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-PD-1 antibody comprises HCDR1 with the amino acid sequence as set forth in SEQ ID NO:50, HCDR2 with the amino acid sequence as set forth in SEQ ID NO:51 and HCDR3 with the amino acid sequence as set forth in SEQ ID NO:52, and, the light chain variable region of the anti-PD-1 antibody comprises LCDR1 with the amino acid sequence as set forth in SEQ ID NO:53, LCDR2 with the amino acid sequence as set forth in SEQ ID NO:54 and LCDR3 with the amino acid sequence as set forth in SEQ ID NO:55;
preferably, the heavy chain variable region of the anti-PD-1 antibody has an amino acid sequence as set forth in SEQ ID NO: 48, and the light chain variable region has an amino acid sequence as set forth in SEQ ID NO: 49.

20. A combination product, which comprises a first product and a second product in separate packages, wherein,
the first product comprises the anti-PVRIG antibody or antigen-binding fragment thereof according to any one of claims 1 to 9 or the conjugate according to claim 13;
the second product comprises at least one anti-TIGIT antibody;
preferably, the combination product further comprises a third product, the third product comprising at least one anti-PD-1 antibody or at least one anti-PD-L1 antibody;
preferably, the first product, the second product and the third product also independently contain one or more pharmaceutically acceptable excipients;
preferably, the combination product further comprises a package insert.

21. The combination product according to claim 20, wherein,
the molar ratio of anti-PVRIG antibody or its antigen-binding fragment to anti-TIGIT antibody is from (1:5) to (5:1);
and / or
the molar ratio of anti-PVRIG antibody or antigen-binding fragment to anti-PD-1 antibody or anti-PD-L1 antibody is from (1:5) to (5: 1).

22. The combination product according to any one of claims 20 to 21, wherein, the anti-TIGIT antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-TIGIT antibody comprises HCDR1 with the amino acid sequence as set forth in SEQ ID NO:42, HCDR2 with the amino acid sequence as set forth in SEQ ID NO:43 and HCDR3 with the amino acid sequence as set forth in SEQ ID NO:44, and, the light chain variable region of the anti-TIGIT antibody comprises LCDR1 with the amino acid sequence as set forth in SEQ ID NO:45, LCDR2 with the amino acid sequence as set forth in SEQ ID NO:46 and LCDR3 with the amino acid sequence as set forth in SEQ ID NO:47;
preferably, the heavy chain variable region of the anti-TIGIT antibody has an amino acid sequence as set forth in SEQ ID NO:40, and the light chain variable region has an amino acid sequence as set forth in SEQ ID NO:41.

23. The combination product according to any one of claims 20 to 22, wherein, the anti-PD-1 antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-PD-1 antibody comprises HCDR1 with the amino acid sequence as set forth in SEQ ID NO:50, HCDR2 with the amino acid sequence as set forth in SEQ ID NO:51 and HCDR3 as set forth in SEQ ID NO:52, and, the light chain variable region of the anti-PD-1 antibody comprises LCDR1 with the amino acid sequence as set forth in SEQ ID NO:53, LCDR2 with the amino acid sequence as set forth in SEQ ID NO:54 and LCDR3 with the amino acid sequence as set forth in SEQ ID NO:55;
preferably, the heavy chain variable region of the anti-PD-1 antibody has an amino acid sequence as set forth in SEQ ID NO: 48, and the light chain variable region has an amino acid sequence as set forth in SEQ ID NO: 49.

24. Use of the antibody or antigen-binding fragment thereof according to any one of claims 1 to 9, or of the conjugate according to claim 13 in the preparation of a medicament for the treatment or prevention of a tumor;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.

25. The antibody or antigen-binding fragment thereof according to any one of claims 1 to 9 or the conjugate according to claim 13 for use in the treatment or prevention of a tumor;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.

26. A method for treating or preventing a tumor, comprising a step of administering to a subject in need an effective amount of the antibody or antigen-binding fragment thereof according to any one of claims 1 to 9 or the conjugate according to claim 13;
preferably, the tumor is one or more selected from the group consisting of colon cancer, melanoma, lung cancer, kidney cancer, endometrial cancer, breast cancer, skin cancer, ovarian cancer, gastric cancer, head and neck cancer, liver cancer, brain tumor, urothelial cancer, bone tumor, cholangiocarcinoma, rectal cancer, pancreatic cancer, cervical carcinoma, multiple myeloma, Hodgkin's lymphoma, non-Hodgkin's lymphoma, B-lymphoma, plasma cell cancer, prostate cancer and testicular cancer;
preferably, the lung cancer is non-small cell lung cancer or small cell lung cancer.
